# EUROPEAN PATENT APPLICATION

(11) **EP 2 674 121 A1**
(43) Date of publication of application: **18.12.2013**
(21) Application number: 12745289.4
(22) Date of filing: 10.02.2012
(51) Int. Cl.: A61B 17/56

(54) **INTERSPINOUS PROCESS SPACING DEVICE**

(30) Priority: 11.02.2011 US 201161441802 P
(71) Applicant: Terumo Kabushiki Kaisha, Tokyo 151-0072 (JP)
(72) Inventor: HATA, Suguru, Ashigarakami-gun, Kanagawa 259-0151 (JP); NAKAGAWA, Yuji, Ashigarakami-gun, Kanagawa 259-0151 (JP); TADA, Yuichi, Ashigarakami-gun, Kanagawa 259-0151 (JP); KINOSHITA, Yasushi, Ashigarakami-gun, Kanagawa 259-0151 (JP)
(74) Representative: Dossmann, Gérard
(86) International application number: PCT/JP2012/053155
(87) International publication number: WO 2012/108531

(57) **Abstract**

Disclosed is a method for dilating between spinous processes wherein a filamentous body provided with an expansion body is percutaneously inserted to locate the expansion body between the spinous processes, a filling material is injected into the expansion body to dilate between the spinous processes, and the expansion body is cut off from the filamentous body to be located between the spinous processes. This method exerts very little burden on the patient.

## Description

### TECHNICAL FIELD

The present invention relates to an expansion device between spinous processes and a method for dilating between spinous processes.

### BACKGROUND

A lumbar spine has a vertebral body as a front half and a vertebral arch as a rear half which are interconnected through a pedicle of vertebral arch, with the vertebral arch formed with processes such as a spinous process, a mamillary process and an accessory process, and normally has a slightly forwardly curved shape. In addition, since adjacent lumbar spines are interconnected through an intervertebral disk, a certain lumbar spine and the adjacent spine, for example, are prevented by an intervertebral disk and a facet joint from getting out of alignment with each other. When a load is repeatedly exerted on the lumbar spine due to sports or the like to cause a fatigue fracture, there would result lumbar spondylolysis in which the lumbar degenerated spondylolisthesis in which it becomes difficult to fix the upper lumbar spine due to degeneration of the intervertebral disk or the shape of the facet joint and a slip-off of the lumbar spines is thereby caused. Further, a severe slip of lumbar spines may cause stenosis of the vertebral canal, possibly leading to intermittent claudication, which is a symptom of lumbar spinal canal stenosis.

In the case of a fracture of a facet joint, such as lumbar degenerative spondylolisthesis and lumbar spondylolysis, there is a high possibility of postoperative instability, which needs fixation (fusion). Therapeutic methods for such a case include fixation of the vertebral bodies by posterior lumbar interbody fusion (PLIF).

The posterior lumbar interbody fusion is said to be an ideal surgical technique, in view of the fact that nerve decompression and interbody fusion can thereby be carried out simultaneously. In this method, after muscle and ligament resection under general anesthesia, bone perforation is conducted and an implant is firmly fixed by screwing.

A patient with lumbar degenerative spondylolisthesis or lumbar spondylolysis should be hospitalized for an average of about 60 days, in general; in addition, this technique inevitably involves general anesthesia. Therefore, this technique cannot be applied to patients with a cardiopathy or a respiratory organ disease or the like. Besides, the resection of muscle or the like and the bone perforation exert heavy burdens on the patient. Further, placement of an implant leads to exertion of a load on the anchoring screw or the adjacent vertebral body, so that there arises a side effect of fracture in the site where the implant is put indwelling.

On the other hand, therapeutic methods commonly used for lumbar spinal canal stenosis have basically been preservative therapies, such as pharmacotherapy using an analgetic agent or antiphlogistic or the like, gymnastics for strengthening abdominal or back muscles or the like, thermotherapy using a hot pack or the like, acupuncture therapy aimed at a pain-eliminating effect, orthotic therapy using a corset or the like, and nerve block therapy. In the case of a gravy patient, however, decompressive laminectomy in which spinal cords and nerve roots are decompressed is performed. In this therapeutic procedure, the back and muscles are incised, and the support structures are removed from the backbone, to expose the rear surface of the spinal column. Then, the part of the vertebral arch covering the back side of the vertebral canal is removed (laminectomy), whereby the thickened yellow ligament is exposed, followed by extraction of the ligament of the thickened yellow ligament. This procedure is carried out under general anesthesia. Normally, the patient should be hospitalized for about five to seven days depending on the age and general conditions of the patient, and, further, it takes a time of from six weeks to three months for the patient to recover from the procedure. This technique, also, inevitably involves general anesthesia and, therefore, cannot be applied to patients with a cardiopathy or a respiratory organ disease or the like. In addition, many patients must be treated for an extended time in rehabilitation facilities in order to recover motility for living independently, and such an extended treatment is a considerable burden on the patients. Besides, the interbody fusion such as posterior lumbar interbody fusion (PLIF) is a technique used also for therapy of intervertebral disk hernia so as to achieve fixation (fusion) between spinous processes or achieve interbody fusion, thereby dilating between the spinous processes.

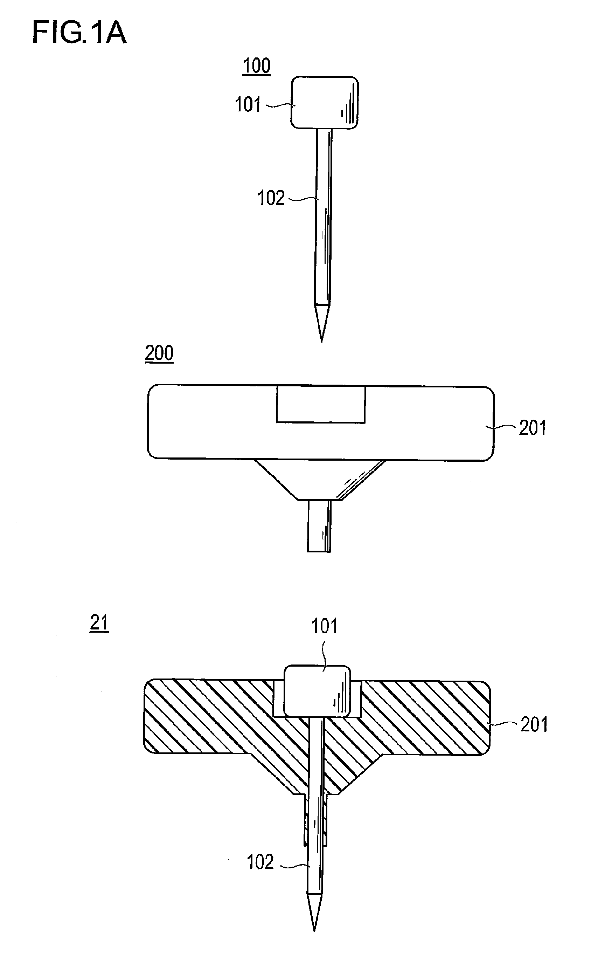

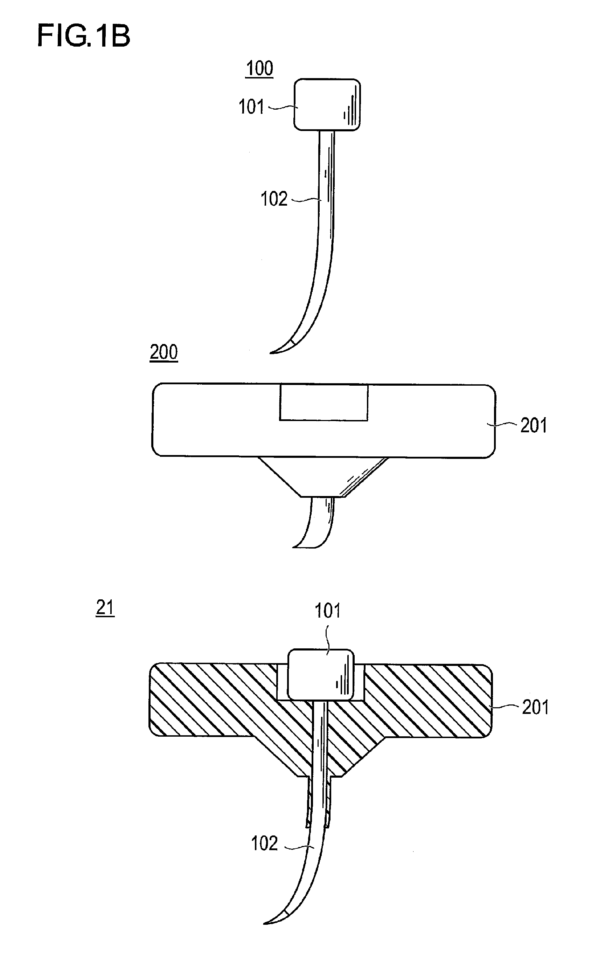

In order to alleviate the burdens exerted on the patients by these techniques, for example, Patent Document 1 (US 2004/0199255 A1) discloses an H-type prosthesis to be embedded between spinous processes. This prosthesis has a pair of clamping members. Specifically, as shown in FIGS. 1, 2 and 4 of the document, one of the clamping members has a linking body projected from a flat elliptical main body, while the other has a non- linking body extended from a flat elliptical main body so as to be attached to the linking body.

### SUMMARY OF INVENTION

The use of the prosthesis according to Patent Document 1 ensures that the burden on the patient due to bone perforation is alleviated and, by embedding the H-type prosthesis between spinous processes, it is possible to dilate between the spinous processes and to achieve interbody fusion. It is still necessary, however, to incise the back and muscles under general anesthesia, so that the patient has to be hospitalized for a long period of time, which is again a considerable burden on the patient.

In order to solve the above-mentioned problems, it is an object of the present invention to provide a puncture-type expansion body device or an expansion device assembly between spinous processes for dilating between spinous processes by which interbody fusion can be achieved without incision of the back and muscles under general anesthesia.

According to an embodiment of the present invention, there is provided a puncture-type expansion body device comprising: a puncture needle provided with a puncture body at a distal end thereof; a balloon-formed expansion body which is expanded by injecting a filling material into the inside thereof; an intermediate connection section which is provided between the puncture needle and the expansion body and by which the expansion body is guided to a part punctured by the puncture needle; and a pressure feed tube body for injecting the filling material into the expansion body.

Further, according to another embodiment of the present invention, there is provided an expansion device assembly between spinous processes comprising: a puncture needle having coaxially an inner needle provided with a needle body which is a solid body having a sharp distal end and an outer needle provided with a tubular body at a distal end thereof, having a lumen which is inserted and passed through the needle body; and a filamentous body provided with an expansion body having a plurality of bulging parts interconnected through a constricted part at the distal end of a pressure feed tube body for feeding out a filling material.

According to the present invention, it is possible to effectively treat cervical spinal disk herniation, cervical spondylosis, ossification of posterior longitudinal ligament, lumbar disk herniation, lumbar spondylolysis, lumbar spondylolisthesis, spinal canal stenosis, atlantoaxial dislocation, chiari malformation-syringomyelia, and tumor of spinal cord, or to effectively mitigate the symptoms of these diseases.

### BRIEF DESCRIPTION OF THE DRAWINGS

[FIG. 1A] FIG. 1A is a schematic view of an embodiment of a puncture needle to be used in the present invention;
[FIG. 1B] FIG. 1B is a schematic view of an embodiment of a puncture needle to be used in the present invention;
[FIG. 2] FIG. 2 is a schematic view of a shears device to be used in the present invention;
[FIG. 3] FIG. 3 is a schematic view of an embodiment of a filamentous body provided with an expansion body to be used in the present invention;
[FIG. 4A] FIG. 4A is a schematic view of an embodiment of a puncture type expansion body device to be used in the present invention;
[FIG. 4B] FIG. 4B is a schematic view of another embodiment of the puncture type expansion body device to be used in the present invention;
[FIG. 5] FIG. 5 is a schematic view of an approach site of a puncture needle, as a patient to whom the method of the present invention is applied is viewed from the rear side;
[FIG. 6] FIG. 6 is a schematic view of the approach site of the puncture needle, as a patient to whom the method of the present invention is applied is viewed in a section perpendicular to the axial direction of the spinal column;
[FIG. 7A] FIG. 7A is a schematic view of a state in which a shears device is inserted, as a patient to whom the method of the present is applied is viewed in side view relative to the axial direction of the spinal column;
[FIG. 7B] FIG. 7B is a schematic view of a condition where incision is performed by a shears device, as a patient to whom the method of the present invention is applied is viewed in side view relative to the axial direction of the spinal column;
[FIG. 8] FIG. 8 is a schematic view of a condition where an expansion body is inserted, as a patient to whom the method of the present invention is applied is viewed in a section perpendicular to the axial direction of the spinal column;
[FIG. 9] FIG. 9 is a schematic view of a condition where an expansion body located between spinous processes is expanded, as a patient to which the method of the present invention is applied is viewed in a section perpendicular to the axial direction of the spinal column;
[FIG. 10] FIG. 10 is a schematic view of a condition where an expanded expansion body is cut off from a filamentous body, as a patient to whom the method of the present invention is applied is viewed from a section perpendicular to the axial direction of the spinal column;
[FIG. 11] FIG. 11 is a schematic view of an expansion body disposed between spinous processes, as a patient to whom the method of the present invention is applied is viewed in a section perpendicular to the axial direction of the spinal column;
[FIG. 12] FIG. 12 is a schematic view of an expansion body located, as a patient to whom the method of the present invention is applied is viewed from the rear side;
[FIG. 13] FIG. 13 is a schematic view of an expansion body expanded between spinous processes, as a patient to whom the method of the present invention is applied is viewed in a section perpendicular to the axial direction of the spinal column;
[FIG. 14] FIG. 14 is a schematic view of a condition where an injection port of an expansion body is sealed off, as a patient to whom the method of the present invention is applied is viewed in a section perpendicular to the axial direction of the spinal column;
[FIG. 15] FIG. 15 is a schematic view of a condition where an expansion body with an injection port sealed off by the method of Example 2 is located between spinous processes, as a patient is viewed in a section perpendicular to the axial direction of the spinal column;
[FIG. 16] FIG. 16 is a schematic view of a condition where an expansion body with an injection port sealed off by the method of Example 2 is located between spinous processes, as a patient is viewed from the rear side;
[FIG. 17] FIG. 17 is a schematic view of a condition where a puncture needle is made to penetrate between spinous processes by the method of Example 3, as a patient is viewed in a section perpendicular to the axial direction of the spinal column;
[FIG. 18] FIG. 18 is a schematic view of a condition where a puncture needle is made to penetrate between spinous processes by the method of Example 4, as a patient is viewed in a section perpendicular to the axial direction of the spinal column; and
[FIG. 19] FIG. 19 is a schematic view of a condition where a puncture needle is made to penetrate between spinous processes by the method of Example 5, as a patient is viewed in a section perpendicular to the axial direction of the spinal column.

### DETAILED DESCRIPTION OF THE PREFERRED EMBODIMENTS

The embodiment of the present invention will be described below. This application is based on U.S. Provisional Patent Application Serial No. 61/441802 filed on February 11, 2010, the entire contents of which are incorporated herein by reference.

A first aspect of the present invention is an expansion device assembly between spinous processes comprising: a puncture needle having coaxially an inner needle provided with a needle body which is a solid body having a sharp distal end and an outer needle provided with a tubular body at a distal end thereof, having a lumen which is inserted and passed through the needle body; and a filamentous body provided with an expansion body having a plurality of bulging parts interconnected through a constricted part at the distal end of a pressure feed tube body for feeding out a filling material.

Thus, by using the expansion device assembly between spinous processes pertaining to the present invention, an expansion body can be located between spinous processes in a percutaneous manner, without incision of skin. The conventional posterior lumbar interbody fusion (PLIF) is a technique which includes putting the patient into prone position under general anesthesia, incising the skin at a lumbar part along a length of about 10 to 15 cm, optionally resecting a spinous process, thereafter resecting a part of a vertebral arch, performing decompression of nerve while observing a microscope, resecting an intervertebral disk, inserting an artificial member prepared by packing a carbon- or titanium-made cage with the patient's own bone, boring a hole in the pedicle of vertebral arch, inserting a screw from the rear side, and attaching a metallic bar to the screw to achieve firm fixation. Therefore, a considerable burden is inevitably exerted on the patient. Besides, such similar techniques as posterolateral fusion (PLF), transforaminal lumbar interbody fusion (TLIF) based on approach through an intervertebral foramen, and interspinal distraction are also not preferable from the viewpoint of QOL (quality of life).

On the other hand, according to the expansion device assembly between spinous processes of the present invention, this is a method that the expansion body is inserted into the interspinal ligament between spinous processes in a percutaneous manner without incision of skin, followed by expanding the expansion body. Thus, this method does not involve a surgical operation and promises very little burden on the patient (namely, the technique is minimally invasive). Accordingly, the expansion device assembly between spinous processes of the present invention is advantageous in that: (a) the method does not involve skin incision; (b) it is possible by the method to effectively treat cervical spinal disk herniation, cervical spondylosis, ossification of posterior longitudinal ligament, lumbar disk herniation, lumbar spondylolysis, lumbar spondylolisthesis, spinal canal stenosis, atlantoaxial dislocation, chiari malformation-syringomyelia, and tumor of spinal cord, or to effectively mitigate the symptoms of these diseases; (c) the method does not need general anesthesia; (d) the method does not involve bone resection; and (e) the method is minimally invasive to the patient. In addition, the method of the present invention is particularly effective for treatment of grave lumbar spondylolysis, lumbar spondylolisthesis, spinal canal stenosis, and lumbar disk herniation.

The structure of the puncture needle of the present invention is a needle having coaxially, an inner needle provided with a needle body which is a solid body having a sharp distal end; and an outer needle provided with a tubular body at a distal end thereof, having a lumen which is inserted and passed through the needle body. Specifically, the puncture needle has an outer needle 200 and an inner needle 100 which are coaxial, as shown in FIG. 1A and FIG. 1B. Specifically, the outer needle 200 of the prevent invention has a tubular body having at least one lumen which is provided with a handle 201, and the tubular body is located at the distal end of the outer needle. The inner needle 100 of the present invention has a needle body 102 which is a solid body having a sharpened point, and is provided with a handle 101 at its proximal portion. The inner needle 100 is located in the lumen which is the inside of the tubular body of the outer needle 200, and the inner needle 100 can be inserted into and pulled out of the outer needle 200. In Figs. 1A and 1B, the handle 101 as a fixing member is equipped at the basal edge of the needle body 102 for the purpose of preventing a needle body 102 at the front edge of the inner needle 200 with going through in relation to the lumen of the outer needle 200. In addition, concave portion capable of housing the fixing member 101 is formed on the handle 201 of the outer needle 200, and the fixing member 101 is set in the concave portion. Such the puncture needle preferably has the structure of the commercially available Verteport Needles made by Stryker Corporation.

The inside diameter of the outer needle of the puncture needle is preferably 0.5 to 9.5 mm, and the outside diameter of the outer needle is preferably 1 to 10 mm. The length in the longitudinal axial direction (the length from the distal end to a proximal portion provided with the handle, here and below) of the outer needle of the puncture needle is preferably 1 to 20 cm.

The diameter of the inner needle of the puncture needle is preferably 0.4 to 9.4 mm. The length in the longitudinal axial direction of the inner needle of the puncture needle is preferably 1.5 to 25 cm.

Examples of the material which can be used to form the puncture needle, the materials for both of the outer needle and the inner needle, include metallic materials such as stainless steel, aluminum or aluminum alloys, titanium or titanium alloys, cobalt-chromium or cobalt-chromium alloys, etc. and various flexible materials such as ethylene-tetrafluoroethylene copolymer (ETFE), polyurethane, polyether-nylon resins, etc.

In addition, the outer surface of the outer needle of the puncture needle is preferably formed with grooves so that echoes are reflected thereon. This ensures that puncture can be made while confirming the position of the puncture needle under echographic observation.

The shape of each of the outer needle and the inner needle may be any of a rectilinear shape, a crescent shape, a fishhook-like shape, and a U-shape. Or, like an injection needle, the outer needle and the inner needle may have a tubular hollow body having a sharp point, with the inside of the tubular form functioning as a lumen.

For example, FIG.1A shows that the needle body 102 of the inner needle and the tubular body of the outer needle are a rectilinear shape, FIG.1B shows that the needle body 102 of the inner needle and the tubular body of the outer needle are a crescent shape, and either front edge of inner needle or outer needle according the present invention may have a nib part.

The outer needle according to the present invention has a sharp point and a tubular hollow body. The outer needle, such as an injection needle, may have a tubular hollow body having a sharp point, with the inside of the tubular form functioning as a lumen.

The puncture needle according to the present invention has a structure including an outer needle having at least one lumen and an inner needle inserted and passed through the lumen, wherein at least either one of the outer needle and the inner needle has a sharp point part. The shape of each of the outer needle and the inner needle may be any of a rectilinear shape, a crescent shape, a fishhook-like shape, and a U-shape. Or, like an injection needle, the outer needle and the inner needle may have a tubular hollow body having a sharp point, with the inside of the tubular form functioning as a lumen.

In the case where the outer needle and the inner needle are rectilinear or crescent-like in shape, a structure may be adopted in which the outer needle and the inner needle are provided coaxially, the outer needle has a tubular body at the distal end thereof, the inner needle is provided inside the lumen of the outer needle and is a solid needle with a sharpened point, and the inner needle can be inserted into and pulled out of the outer needle. In addition, where the outer needle and the inner needle are rectilinear or crescent-like in shape, it suffices that either the outer needle or the inner needle has a sharp point part at the distal end thereof.

In this case, example of the material which can be used to form the puncture needle, the materials for the outer needle and the inner needle, include metallic materials such as stainless steel, aluminum or aluminum alloys, titanium or titanium alloys, cobalt-chromium or cobalt-chromium alloys, etc., and various flexible materials such as ethylene-tetrafluoroethylene copolymer (ETFE), polyurethane, polyether-nylon resin, etc.

In the case where the outer needle and the inner needle are fishhook-shaped or U-shaped, it is preferable that the outer needle has a sharp point part, is fishhook-shaped or U-shaped and has a lumen therein, while the inner needle is inserted in the lumen of the outer needle, and the inner needle is an elastic body which can be bent to a certain extent. Besides, where the outer needle and the inner needle are fishhook-shaped or U-shaped, it is preferable that the outer needle has a sharp point part at the distal end thereof.

In this case, as for materials for the puncture needle, the outer needle is preferably formed of a metallic material such as stainless steel, aluminum oraluminum alloys, titanium or titanium alloys, cobalt-chromium or cobalt-chromium alloys, etc., whereas the inner needle is preferably formed of any of various flexible materials such as ethylene-tetrafluoroethylene copolymer (ETFE), polyurethane, polyether-nylon resin, etc.

In addition, where the puncture needle is rectilinear in shape, the length in the longitudinal axial direction of the outer needle is preferably 1 to 20 cm, and the length in the longitudinal axial direction of the inner needle of the puncture needle is preferably 1.5 to 25 cm.

Where the puncture needle is fishhook-shaped or U-shaped, the length from the distal end of the outer needle to the proximal end where the handle is provided is preferably 3 to 20 cm, and the length from the distal end of the inner needle to the proximal end where the handle is provided is preferably 3.5 to 25 cm.

The number of lumens, the diametral size and the like of the puncture needle according to the present invention may be appropriately determined by the clinician, taking into account the ease of access to a target site, the patient's body shape and the like.

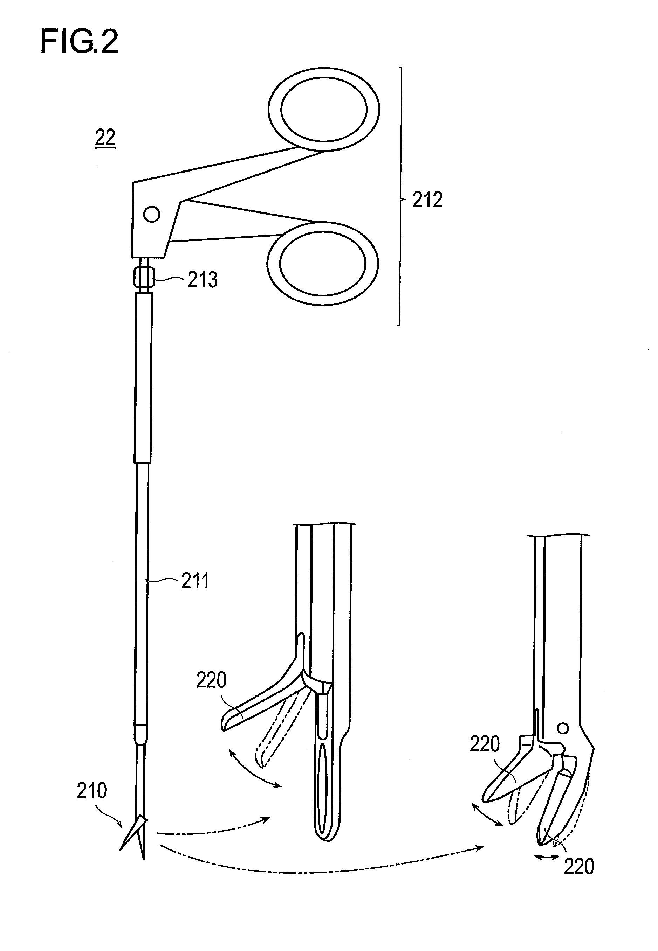

A shears device according to the present invention has a blade part capable of dissecting a ligament at the front edge and an operating part for operating the blade part at the basal edge, and the blade part is capable of inserting into the lumen of the outer needle. Specifically, as shown in the Fig. 2, the blade part 201 at the front edge divides dichotomously and the dichotomous blade part 201 is linked with the operating part 212 via the shaft 211, thereby the blade parts 210 can gate (like long scissors) by being operated with the operating part 212 at the basal edge. For instance, Fig. 2 (A) is a magnified example of the blade part of the shears device, where only one of the dichotomous blade parts 210 is a blade 220, which is operated so as to rotate up to about 180 degrees around the basal edge as the center. In addition, Fig. 2 (B) is another magnified example of the blade part of the shears device, where both of the dichotomous blade parts are blades, which are operated so as to rotate up to about 180 degrees around the basal edge as the center. Further, in the shears device of the present invention, the direction of the blade part can be freely adjusted around the longitudinal direction of the shaft by rotating the dial 213, which is equipped at the operating part and capable of rotating freely. For instance, the structure of a rongeur is preferably used as such shears device (FIG. 2).

In the case where the puncture needle is crescent-shaped, fishhook-shaped or U-shaped, it is preferable for the shears device also to be crescent-shaped, fishhook-shaped or U-shaped in conformity with the shape of the puncture needle.

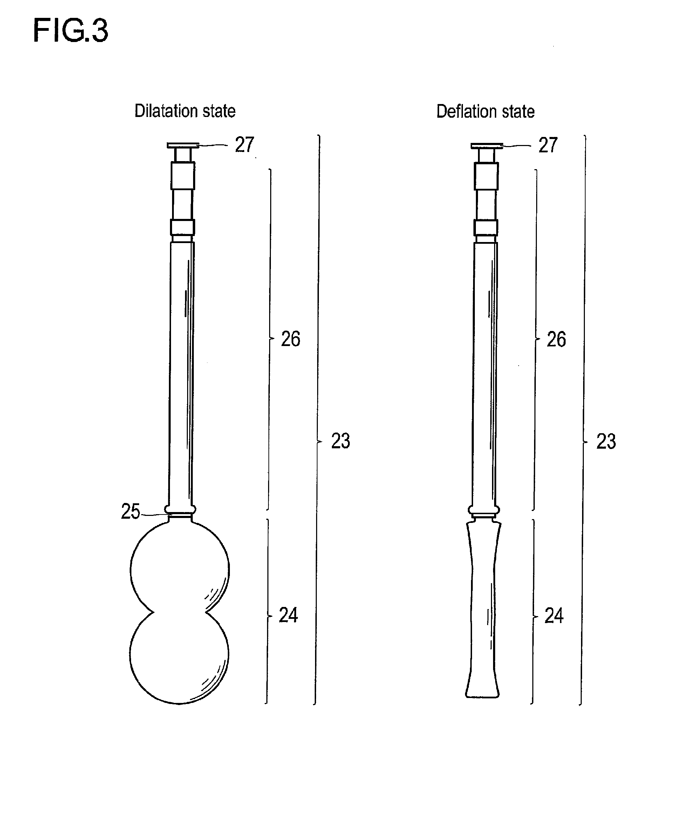

The filamentous body according to the present invention, preferably, has a structure which includes an expansion body having a plurality of bulging parts interconnected through a constricted part, and a pressure feed tube body for feeding out a filling material, the expansion body being provided with an injection port, the injection port communicating with the pressure feed tube body, and the pressure feed tube body being in connection with a pressure feeder such as a syringe or a pump (see FIG. 3). In addition, preferably, the expansion body is balloon-like in shape, is a tubular body when contracted, and has a structure which includes a plurality of bulging parts interconnected through a constricted part when expanded. FIG. 3 is a schematic view of a filamentous body 23 having an expansion body at the distal end thereof. The filamentous body 23 has a structure including the expansion body 24, the pressure feed tube body 26 for feeding out a filling material through the injection port 25 of the expansion body 24, and the pressure feeder 27 in connection with the pressure feed tube body.

The material of the expansion body is not particularly restricted, insofar as the expansion body can expand when a filling material is injected thereinto and the expansion body material is durable to external pressures attendant on movements of vertebral bodies as well as tissues around the expansion body, such as interspinal ligament, yellow ligament and spinous processes. Preferable examples of the material include polyvinyl chloride, polyurethane, polyurethane elastomer, styrene-ethylene-butylene-styrene copolymer (SEBS), styrene-ethylene-propylene-styrene copolymer (SEPS), ethylene-vinyl acetate copolymer (EVA), nylon and the like polyamide resins and polyamide elastomers, polyethylene terephthalate (PET) and the like polyester resins and polyester elastomers, polyethylene and the like olefin resins, rubbers, silicone elastomers, fluoro-rubbers, and fluoro-resins.

In addition, the expansion body may have such a structure that air present in the expansion body or the pressure feed tube body passes therethrough when the filling material is injected. For instance, the whole part or a part of the expansion body may be composed of a porous body such as woven or knitted bodies formed from PET fibers or the like, stretched (oriented) PTFE or ceramic filters, hollow fibers, etc. Or, the expansion body may be formed in the surface thereof with one minute hole or a plurality of minute holes by use of laser or a needle or the like.

The axial length of the expansion body when contracted is preferably 1 to 10 cm, and the axial length when expanded is preferably 1 to 12 cm.

The shape of the expansion body is not specifically restricted, insofar as it has a plurality of bulging parts interconnected through a constricted part. Preferred is a shape which has a pair of bulging parts communicating with each other through a connection part and which clamps an interspinal ligament in a gripping manner. More preferred is a structure which is rotationally symmetric about the longitudinal axial direction of the expansion body. Further preferred is a dumbbell-like shape, a wheels-like shape (H-shape) or the like.

In addition, the maximum length of section of the bulging part in the expanded state of the expansion body is preferably 1 to 10 cm.

The axial length of the constricted part of the expansion body when contracted is preferably 0.1 to 8 cm, and the axial length of the constricted part when expanded is preferably 0.1 to 10 cm.

The maximum length of section of the constricted part in the expanded state of the expansion body is preferably 0.2 to 5 cm.

The material thickness of the expansion body when contracted is preferably 0.01 to 1 mm.

The maximum diameter of the injection port of the expansion body is preferably 0.5 to 9.8 mm.

The diametral size, length, shape and the like of the pressure feed tube body are appropriately selected according to the shape and size of the expansion body used, the size of the injection port, etc.

It is preferable for the expansion body to be folded compactly in relation to the longitudinal axis thereof when contracted. This ensures that the inside and outside diameters of the puncture needle in which to insert and locate the expansion body can be set as small as possible, resulting in that the method according to the present invention is minimally invasive to the patient.

Besides, the expansion body desirably has radiopaque properties. As the method for imparting radiopaque properties to the expansion body, known methods can be used appropriately. For instance, in the case where the expansion body is formed by blow molding from a thermoplastic resin such as a polyamide elastomer, a method may be adopted in which a radiopaque material such as barium sulfate is preliminarily kneaded into the thermoplastic resin and the expansion body is molded from the thus kneaded resin. In the case where the expansion body is a body woven from PET fibers, a method may be adopted in which a radiopaque material such as barium sulfate is preliminarily kneaded into PET resin and the PET fibers spun from the thus kneaded resin are used to form the whole part of a part of the expansion body. With the radiopaque properties thus imparted to the expansion body, it becomes possible to radioscopically confirm the condition in which the expansion body is located between spinous processes.

The filling material according to the present invention is not specifically restricted, insofar as it has elasticity or hardness on such a level that when located between spinous processes it can hold the mutually separated state of the expanded spinous processes. Specifically, the filling material is not particularly limited, insofar as it is a safe material which hardens (cures) at a temperature of about 60°C or below, particularly at a temperature around the body temperature. As the filling material, for example, hardened materials being fluid at the time of injection and hardening after the injection and materials being fluid at the time of injection and non-hardening after the injection can both fluids be used preferably. In addition, it is not necessary for the filling material to be a fluent material as a flowing body (hardened materials being fluid at the time of injection and non-hardening after the injection), and a non-fluid material can be used as the filling material besides the fluids. Further, a combination of a fluid material and a non-fluid material can be properly used as the filling material in the present invention.

The hardened materials being fluid at the time of injection and hardening after the injection and the materials being fluid at the time of injection and non-hardening after the injection, preferably, have at least one of the following characteristics: (a) to be safe to the patient; (b) to cause little or no damage to the tissues; (c) to harden at a temperature near the patient's body temperature (about 35 to 42°C); (d) to be free of contraction or expansion and be able to maintain the shape upon hardening; (e) to harden within 1 to 60 minutes, preferably 5 to 30 minutes, and more preferably 10 minutes, after the injection; (f) to permit use of water, buffers, physiological saline, contrast agents, and oils and fats such as olive oil and castor oil, as a solvent therefor.

Specific examples of the hardened material being fluid at the time of injection and hardening after the injection (the material will hereafter be referred to as "hardening material") include: (i) two-part type crosslinking polymers; (ii) hot melt adhesives; (iii) urethane elastomers; (iv) photo-curing resins; (v) acrylic resins; (vi) bone cements; and (vii) solutions which are crystallized in response to an external stimulus.

The two-part type crosslinking polymers of (i) above are each preferably a combination of an aromatic diepoxide resin or aliphatic diepoxide resin with an amine compound or a combination of a liquid polyorganosiloxane having a reactive group with a crosslinking agent and a curing catalyst.

The aromatic diepoxide resin is not particularly restricted, and examples thereof include diglycidyl ether of bisphenol A, and diglycidyl ether of bisphenol F. The aliphatic diepoxide resin is not specifically restricted, and examples thereof include diepoxide resins which are alkane diols of glycidyl ether, such as pentanediol of glycidyl ether, butanediol of glycidyl ether, and propanediol of glycidyl ether. The amine compound is not particularly limited, and examples thereof include 1,3-diaminopropane, diethylenetriamine, triethylenetetramine, N-aminoethylpiperazine nonyl/phenol, and dialkylamine compounds represented by the formula: H₂N-(R-NH)ₓ-R-NH₂, where groups R are independently straight chain or branched alkyl groups of 2 to 10 carbon atoms, preferably 2 to 5 carbon atoms, and x is 0, 1 or 2. Among these hardening materials of (i), preferred are those hardening materials which are each composed of about 60 to 80 wt.%, preferably about 65 to 75 wt.%, of an aromatic diepoxide resin and/or an aliphatic diepoxide resin and about 20 to 40 wt.%, preferably about 25 to 35 wt.%, of an amine compound [the total amount of the aromatic diepoxide resin and/or the aliphatic diepoxide resin and the amine compound is 100 wt.%]. Particularly preferred are those hardening materials which are each composed of about 45 to 52 wt.% of an aromatic diepoxide resin, about 19 to 23 wt.% of an aliphatic diepoxide resin, about 20 to 29 wt.% of a dialkylamine compound, and about 4 to 9 wt.% of N-aminoethylpiperazine nonyl/phenol [the total amount of the aromatic diepoxide resin, the aliphatic diepoxide resin, the dialkylamine compound and N-aminoethylpiperazine nonyl/phenol is 100 wt.%].

Examples of the polyorganosiloxane include those which have a methyl group, a vinyl group, a hydroxyl group, a ketone group or the like as a functional group on a backbone siloxane, among which addition-type polyorganosiloxanes having a vinyl group are preferred. Besides, as the crosslinking agent, there is used a polysiloxane having a hydrogen group, or the like. As the catalyst, there is used a platinum compound, an organometallic compound, or the like. Furthermore, a reaction controller, a reinforcement silica and other fillers and/or additives or the like may be contained in the polyorganosiloxane composition.

Examples of the hot melt adhesives of (ii) above include combinations of water with a material capable of being hardened by reaction with water, and adhesives based on EVA (ethylene-vinyl acetate copolymer), PO (polyolefin), PA (polyamide), SR (synthetic rubber), ACR (acryl), or PUR (polyurethane; moisture-curing type).

The material capable of being hardened by reaction with water is not specifically restricted, insofar as the reaction (hardening) starts in the presence of water. Specifically, cyanoacrylate monomers are preferably used as this type of material. In this case, upon contact with water, the cyanoacrylate monomer is polymerized to be polycyanoacrylate. Specific examples of the cyanoacrylate monomers include alkyl and cycloalkyl α-cyanoacrylates such as methyl α-cyanoacrylate, ethyl α-cyanoacrylate, propyl α-cyanoacrylate, butyl α-cyanoacrylate, pentyl α-cyanoacrylate, hexyl α-cyanoacrylate, heptyl α-cyanoacrylate, octyl α-cyanoacrylate, cyclohexyl α-cyanoacrylate, etc.; alkenyl and cycloalkenyl α-cyanoacrylates such as allyl α-cyanoacrylate, methacryl α-cyanoacrylate, cyclohexenyl α-cyanoacrylate, etc.; alkynyl α-cyanoacrylates such as propargyl α-cyanoacrylate, etc.; aryl α-cyanoacrylates such as phenyl α-cyanoacrylate, toluyl α-cyanoacrylate, etc.; α-cyanoacrylates containing a hetero atom, such as methoxyethyl α-cyanoacrylate, ethoxyethyl α-cyanoacrylate, furfuryl α-cyanoacrylate, etc.; α-cyanoacrylates containing a silicon atom, such as trimethylsilylmethyl α-cyanoacrylate, trimethylsilylethyl α-cyanoacrylate, trimethylsilylpropyl α-cyanoacrylate, dimethylvinylsilylmethyl α-cyanoacrylate, etc. These α-cyanoacrylates may be used either singly or in the form of a mixture of two or more of them. Among these cyanoacrylate monomers, preferred ones include cyclohexyl α-cyanoacrylate, heptyl α-cyanoacrylate, octyl α-cyanoacrylate, and cyclohexenyl α-cyanoacrylate. A polymerized cured product of such a cyanoacrylate in which the ester side chain length is large is flexible, and, therefore, it causes little damage to the tissues. Incidentally, in the case of using the material capable of hardening through reaction with water, a plasticizer may further be used in addition to the material. The use of a plasticizer renders the polymerized hardened product more flexible, whereby damage to the tissues can be restrained or prevented more assuredly.

The urethane elastomers of (iii) above are preferably polymers which are derived from a polyol and an aromatic polyisocyanate.

Examples of the polyol here include polyoxypropylene glycol, polyoxypropylene triol, polyoxyethylene polyoxypropylene glycol, polyoxyethylene polyoxypropylene triol, polyoxypropylene tetraol, polyoxytetramethylene glycol, polyethylene adipate, polybutylene adipate, polyhexamethylene adipate, polyneopentyl adipate, polyethylenepropylene adipate, polyethylenebutylene adipate, polybutylenehexamethylene adipate, polydiethylene adipate, poly(polytetramethylene ether) adipate, polyethylene azelate, polyethylene sebacate, polybutylene azelate, polybutylene sebacate, polyethylene terephthalate, polycaprolactone diol, polycaprolactone triol, polycarbonate diol, and mixtures of two or more of them.

Examples of the aromatic polyisocyanate include 1,4-phenylene diisocyanate, toluene diisocyanate (TDI), diphenylmethane-2,4'- or 4,4'-diisocyanate (MDI), naphthalene-1,5-diisocyanate, 3,3'-dimethyldiphenylmethane-4,4'-diisocyanate, o-tolidine diisocyanate, crude TDI, polyphenylmethane polyisocyanate (crude MDI), their denatured products (denatured products obtained from them by introduction of a carbodiimide group, uretdione group, uretonimine group, urea group, biuret group, isocyanurate group, urethane group or the like), and mixtures of two or more of them.

Besides, the applicable methods here include those known methods in which predetermined amounts of a polyol and an aromatic polyisocyanate are brought into reaction with each other by a one-shot process or a multi-stage process, in the presence of a known urethanation catalyst, if necessary.

Examples of the photo-curing monomers of (iv) above include acrylates, methacrylates, and ethylenically unsaturated carboxylic acids. Besides, a polymerization accelerator, a crosslinking agent, a photo-polymerization initiator or the like can be used, as required, together with the photo-curing monomer(s).

Examples of the acrylates include methyl acrylate, ethyl acrylate, propyl acrylate, butyl acrylate, hexyl acrylate, 2-ethylhexyl acrylate, cyclohexyl acrylate, benzyl acrylate, dimethylaminoethyl acrylate, hydroxyethyl acrylate, hydroxypropyl acrylate, and glycidyl acrylate. Examples of the methacrylates include methyl methacrylate, ethyl methacrylate, propyl methacrylate, butyl methacrylate, hexyl methacrylate, 2-ethylhexyl methacrylate, cyclohexyl methacrylate, benzyl methacrylate, dimethylaminoethyl methacrylate, hydroxyethyl methacrylate, hydroxypropyl methacrylate, and glycidyl methacrylate. As the ethylenically unsaturated carboxylic acid, there are preferably used such monocarboxylic acids as acrylic acid, methacrylic acid, and crotonic acid. Besides, such dicarboxylic acids as maleic acid, fumaric acid, and itaconic acid, and their anhydrides and half-esters can also be used.

Examples of the polymerization accelerator which can be used here include reducing agents such as sodium hydrogensulfite, sodium sulfite, Mohr's salt, sodium hydrogenpyrosulfite, formaldehyde sodium sulfoxylate, ascorbic acid, etc.; and amine compounds such as ethylenediamine, sodium ethylenediaminetetraacetate, glycine, N,N,N',N'-tetramethylethylenediamine, etc.; which may be used either singly or in combination of two or more of them.

When a polymerization accelerator is used, it is preferably added in a concentration in the solution of 0.01 to 10 wt.%, more preferably 0.02 to 1 wt.%.

The photo-polymerization can, for example, be carried out by preliminarily adding a photo-curing initiator to the solution and irradiating the polymerizable monomer(s) with UV rays.

Examples of the photo-polymerization initiator to be used here include acetophenone, 2,2-diethoxyacetophenone, p-dimethylaminoacetophenone, p-dimethylaminopropiophenone, p-dimethylaminopropiophenone, benzophenone, p,p'-dichlorobenzophenone, p,p'-bisdiethylaminobenzophenone, Michler's ketone, benzil, benzoin, benzoin methyl ether, benzoin isopropyl ether, benzoin n-propyl ether, benzoin isobutyl ether, benzyldimethylketal, 1-hydroxy-cyclohexyl phenyl ketone, tetramethylthiuram monosulfide, thioxanthone, 2-chlorothioxanthone, 2,4-dimethylthioxanthone, 2,2-dimethylpropioyl diphenylphosphine oxide, 2-methyl-2-ethylhexanoyldiphenylphosphine oxide, 2,6-dimethylbenzoyldiphenylphosphine oxide, 2,6-dimethoxybenzoyldiphenylphosphine oxide, 2,4,6-trimethylbenzoyldiphenylphosphine oxide, bis(2,6-dimethoxybenzoyl)-2,4,4-trimethylpentylphosphine oxide, 2,3,6-trimethylbenzoyl-diphenylphospine oxide, bis(2,3,6-trimethylbenzoyl)-phenylphosphine oxide, 2,4,6-trimethoxybenzoyldiphenylphosphine oxide, 2,4,6-trichlorobenzoyldiphenylphosphine oxide, 2,4,6-trimethylbenzoylnaphthyl phosphonate, p-dimethylaminobenzoic acid, p-diethylaminobenzoic acid, azobisisobutyronitrile, 1,1'-azobis(1-acetoxy-1-phenylethane), benzoin peroxide, and di-tert-butyl peroxide. These can be used either singly or in combination of two or more of them.

The wavelength of the UV rays is preferably 200 to 450 nm. The dose in irradiation with the UV rays is preferably 100 to 2000 mJ/cm², more preferably 500 to 1500 mJ/cm².

The crosslinking agent to be used in the present invention is appropriately selected according to the kind(s) of the polymerizable monomer(s) used and the polymerization method adopted. The crosslinking agent widely includes those which can introduce a three-dimensional crosslink structure into the polymerizable monomer(s) by polymerizable double bonds. Specific examples of the crosslinking agent include divinyl compounds such as N,N'-methylenebis(meth)acrylamide, N,N'-(1,2-dihydroxyethylene)-bis(meth)acrylamide, diethylene glycol di(meth)acrylate, (poly)ethylene glycol di(meth)acrylate, triethylene glycol di(meth)acrylate, propylene glycol di(meth)acrylate, trimethylolpropane di(meth)acrylate, trimethylolpropane tri(meth)acrylate, pentaerythritol di(meth)acrylate, pentaerythritol tri(meth)acrylate, pentaerythritol tetra(meth)acrylate, (poly)propylene glycol di(meth)acrylate, glycerine tri(meth)acrylate, glycerine acrylate methacrylate, ethylene oxide-modified trimethylolpropane tri(meth)acrylate, pentaerythritol tetra(meth)acrylate, dipentaerythritol hexa(meth)acrylate, etc.; triallyl cyanurate, triallyl isocyanurate, triallyl phosphate, triallylamine, poly(meth)acryloxyalkanes, (poly)ethylene glycol diglycidyl ether, glycerol diglycidyl ether, ethylene glycol, polyethylene glycol, propylene glycol, glycerine, pentaerythritol, ethylenediamine, polyethyleneimine, glycidyl (meth)acrylate, triallyl isocyanurate, trimethylolpropane di(meth)acryl ether, tetraallyloxyethane, glycerol propoxytriacrylate, etc. These crosslinking agents can be used either singly or in combination of two or more of them.

Besides, the solvent is appropriately selected according to the reaction conditions used. For example, organic solvents such as alcohols, THF, hexane, benzene and toluene may be used, and aqueous solutions may also be adopted.

Examples of the acrylic resins of (v) above include those obtained by polymerization, according to known methods, of such monomer(s) as methyl (meth)acrylate, ethyl (meth)acrylate, butyl (meth)acrylate, n-hexyl (meth)acrylate, cyclohexyl (meth)acrylate, 2-ethylhexyal (meth)acrylate, n-octyl (meth)acrylate, nonyl (meth)acrylate, decyl (meth)acrylate, (meth)acrylic acid, glycidyl (meth)acrylate, vinyl acetate, styrene, α-methylstyrene, (meth)acrylamide, and (meth)acrylonitrile.

The bone cements of (vi) above are prepared, for example, by mixing a powder such as polymethyl methacrylate, methyl methacrylate-styrene copolymer, benzoyl peroxide, barium sulfate or the like with a solvent such as methyl methacrylate, N,N-dimethyl-p-toluidine, hydroquinone, etc. Or, alternatively, dental cements and the like can also be used. The bone cements are prepared, for example, by mixing 2-hydroxyethyl methacrylate (HEMA), 4-methacryloxyethyl trimellitate (4-MET), penta(methacryloxyethyloxy)cyclophosphazene monofluoride (PEM-F), a polymerization inhibitor, or a polymerization initiator, with a paste of 1,3-butylene dimethacrylate, sulfinic acid salt, a tertiary amine, a polymerization inhibitor or the like.

Examples of the solutions which are crystallized in response to an external stimulus of (v) above include aqueous solutions prepared by dissolving sodium acetate, sodium chloride or the like. Examples of the external stimulus include physical shocks, heat, light, electricity, and ultrasonic wave.

Examples of the materials which are fluid at the time of injection and not hardened after the injection include: (xi) liquids (physiological saline, contrast agents, medicinal liquids, etc.); (xii) dilatant fluids which are materials varied in hardness according to pressurization speed; (xiii) gels (gelatin, agar-agar, starch, etc.); (xiv) silicone sealants; (xv) biomaterials or bio-absorbable materials; and (xvi) slimes (mixture of polyvinyl alcohol and boric acid).

Examples of the liquids of (xi) above include: physiological saline; contrast agents; prostaglandin E1 preparation; steroid medicines; steroidal antiinflammatory drugs (NSAIDs); antiphlogistic; analgetic; anesthetic; and buffers such as carbonate buffer, phosphate buffer, acetate buffer, HEPES buffer, TRIS buffer, etc. Incidentally, as the contrast agent, those which will be described later can be used suitably.

Examples of the dilatant fluids which are materials varied in hardness according to pressurization speed of (xii) above include metallic powders such as lead powder, etc., and silicone putties (denatured products obtained by adding boron to methyl silicone rubber).

Examples of the gels of (xiii) above include gelatin, agar-agar, starch, sacran, starch- and cellulose-based gels prepared by graft polymerization or carboxymethylation, polyacrylate-based gels, polyvinyl alcohol-based gels, polyacrylamide-based gels, polyoxyethylene-based gels and the like synthetic polymer-based gels, sodium polyacrylate, hydrogel and the like water-absorbing materials. These gels can be used after appropriate water absorption therein by use of water, physiological saline or the like.

Examples of the silicone sealants of (xiv) above which can be used here include such known ones as normal temperature curable oxime type, acetic acid type, and alcohol type silicone sealants. As a primer mixed in the silicone sealant, there can be used ethyl acetate, n-hexane, isopropyl alcohol, silicon dioxide, ethylbenzene, xylene, etc., whereby the adhesiveness, curability and/or hardness of the silicone sealant can be controlled.

Examples of the biomaterials or bio-absorbable materials of (xv) above include such known materials as blood, bones, muscles, other cells, their powders, solutions prepared by use of protease or the like, polylactic acid, collagen, polyglycolic acid, polycaprolactone, polyhydroxybutyric acid, polydioxanone, Plastarch Material, zein, polydioxane, polylactic acid-glycolic acid copolymer, methyl cellulose, polysaccharide, etc. Or, solutions prepared by dissolving these materials by known methods can also be used.

Incidentally, the method for polymerization of the above-mentioned monomeric components of (i) to (vii) is not particularly restricted; for example, such methods as polymerization in solvent, bulk polymerization, etc. can be adopted. Besides, in the case where the hydrophilic polymer in the present invention is a block copolymer or a graft copolymer, non-limitative examples of the polymerization method include living polymerization, polymerization carried out using a macromonomer, polymerization carried out using a polymeric polymerization initiator, and polycondensation.

The amount of the filling material injected into the expansion body according to the present invention is appropriately selected by the surgeon according to such factors as the patient's weight and the expansion body used. For example, the filling material is used in an amount of 1 to 180 mL.

Besides, it is preferable to add a contrast agent to the above-mentioned filling material so as to impart contrast properties to the filling material. As the contrast agent, those which will be described later can be used. This makes it possible to radioscopically confirm the manner in which the expansion body is being filled with the filling material and the expanded state of the expansion body.

Further, as the filling material in the present invention, not only the above-mentioned fluid materials but also non-fluid materials can be used. Examples of the non-fluid filling material include a linear or coil-shaped body of metal or polymer, and granular bodies of metal, polymer or ceramic. Examples of the material which can be used to form the linear or coil-shaped body include stainless steel, nickel-titanium, nickel-titanium alloys, platinum, platinum alloys, polyethylene, PET, polyether-ether ketone (PEEK), polyamides, polylactic acid (PLA), and lactic acid-glycolic acid copolymer (PLGA). The shape of the linear or coil-shaped body is preferably rectilinear, spring-like, chain-like, stent-like, honeycomb-like, or the like. A specific shape may be preliminarily memorized in the linear or coil-shaped body. In the case where the non-fluid filling material is a coil-shaped body, a bending part of the coil can be controlled by changing the pitch of the coil. The linear or coil-shaped bodies may be used either singly or in combination of a plurality of them; in the case of using a plurality of linear or coil-shaped bodies, those which are different in material, shape, or physical properties may be used together.

Examples of the material which can be used to form the granular bodies include stainless steel, aluminum, aluminum alloys, titanium, titanium alloys, tungsten, tungsten alloys, polyethylene, PET, PEEK, polyamides, polyacrylamide, polyvinyl alcohol, polyurethane, ethylene-vinyl acetate copolymer (EVA), PLA, PLGA, calcium phosphate, hydroxyapatite, silica gel, glass, collagen, gelatin, decalcified bone, and autologous bone.

The surfaces of the non-fluid filling material may be rugged by processing, and may be coated with a water-swellable polymer such as polyacrylamide and polyvinyl alcohol.

In addition, the non-fluid filling material may be magnetic.

These non-fluid filling materials may be singly used to fill the expansion body, or may be used together with a fluid filling material to fill the expansion body as discussed previously. In the case where the non-fluid filling material is used together with a fluid filling material to fill the expansion body, the filling of the expansion body with the non-fluid filling material may be conducted before, after, or simultaneously with the filling of the expansion body with the fluid filling material. In the case where the expansion body is filled with the non-fluid filling material before filled with the fluid filling material, the expansion body preliminarily filled with the non-fluid filling material may be put to use, and the non-fluid filling material may be fixed to the inside of the expansion body. Besides, in the case where the non-fluid filling material is a linear or coil-shaped body, a part where a distal portion of the linear or coil-shaped body can be contained, fitted or fixed may preliminarily installed in the inside of the expansion body.

The non-fluid filling material can suppress the fracture of the expansion body after becoming locating by being used together with a coil to improve the stress at rupture. In addition, heat evolution can suppress when a heat-evoluting material such as bone cement is used as the filler.

A second aspect of the present invention is a puncture-type expansion body device comprising: a puncture needle provided with a solid puncture body at a distal end thereof; a balloon-formed expansion body which is expanded by injecting a filling material into the inside thereof; an intermediate connection section which is provided between the puncture needle and the expansion body and by which the expansion body is guided to a part punctured by the puncture needle; and a pressure feed tube body for injecting the filling material into the expansion body. In addition, the pressure feed tube body may be in connection with a pressure feeder such as a plunger of a syringe or a pump.

Thus, by using the puncture-type expansion body device of the present invention, the expansion body is inserted into the interspinal ligament between spinous processes in a percutaneous manner without incision of skin, followed by expanding the expansion body. Thus, this technique does not involve a surgical operation and promises very little burden on the patient. Accordingly, like as the expansion device assembly between spinous processes mentioned above, the puncture-type expansion body device has advantage in (a) to (e) above, and also the method of the present invention is particularly effective for treatment of grave lumbar spondylolysis, lumbar spondylolisthesis, spinal canal stenosis, and lumbar disk herniation.

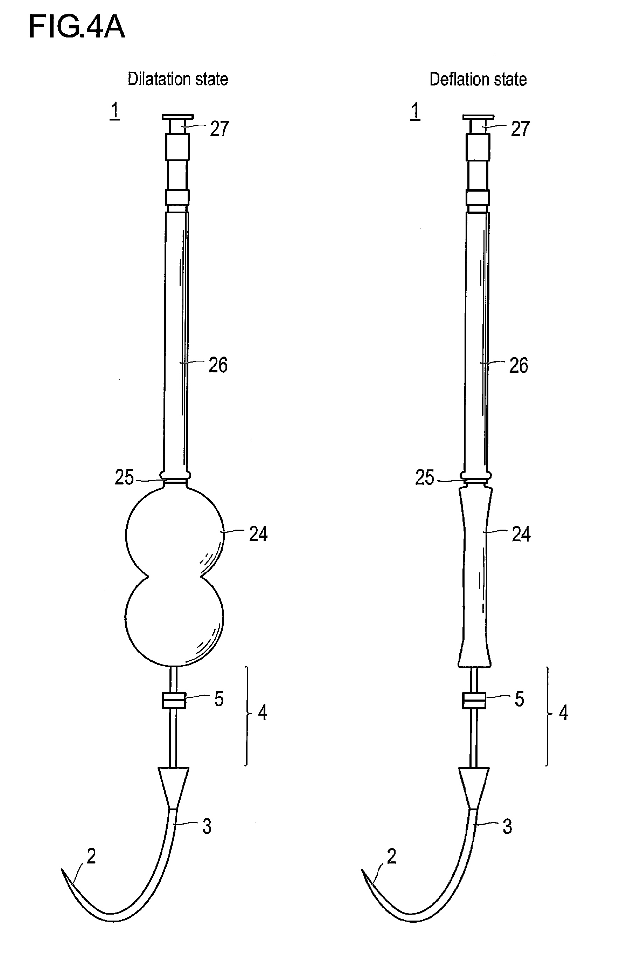

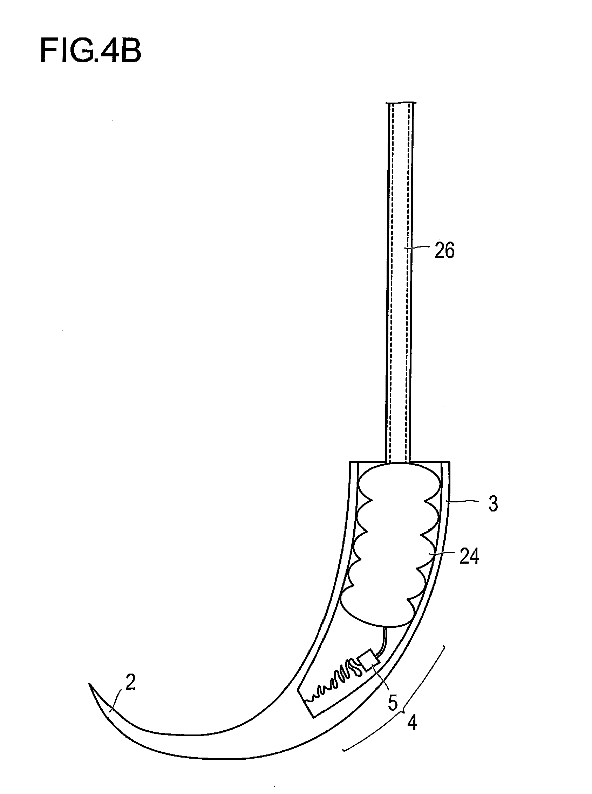

Fig. 4 is a pattern diagram of the puncture-type expansion device of the present invention. The puncture-type expansion device 1 has a structure including a puncture needle 3 with a solid puncture body 2 at the front edge, an intermediate connection section 4 with one edge is equipped to the puncture needle and the other is equipped to an expansion body, a joint 5 formed on the central part of the intermediate connection section 4, the expansion body 24, a pressure feed tube body 26 for injecting a filling material via the inlet 25 of the expansion body 24, and a pressure feeder 27 connected to the pressure feed tube body. In the meantime, the joint is optionally formed. The expansion body is a hollow balloon with an internal space and there is a closed system except for the inlet 25, thereby the lumen inside the pressure feed tube body 26 and the inside of the expansion body are interconnected.

Accordingly, a filling material injected by the pressure feeder flows through the pressure feed tube body into the internal space of the expansion body to inflate the expansion body (left figure of Fig. 4A; the expansion body is inflated). Inversely, the expansion body can be shrunk by aspirating the filling material injected into the inner space of the expansion body (right figure of Fig. 4A; the expansion body is shrunk).

In addition, in the puncture-type expansion device of the present invention, the puncture needle and the expansion body are integrated through the intermediate connection section. Therefore, by adjusting the sliding of the puncture needle or the intermediate connection section and the pressure feed tube body, it is possible to guide the positioning of the expansion body. Accordingly, an axial-directionally central portion of the expansion body can be easily located between the spinous processes.

In addition, the intermediate connection section is a linking body which is attached to the puncture needle at one end thereof and to the expansion body at the other end thereof and by which the puncture needle and the expansion body are interconnected. The intermediate connection section may be provided with a joint. Besides, the puncture needle and the expansion body may be directly interconnected through the intermediate connection section.

The maximum diameter of the puncture needle is preferably 1 to 10 mm, and the length in the longitudinal axial direction (the length from the distal end to a proximal portion connected with the intermediate connection section, here and below) of the puncture needle is preferably 1 to 20 cm.

For instance, examples of the material which can be used to form the puncture needle include metallic materials such as stainless steel, aluminum, aluminum alloys, titanium, titanium alloys, cobalt-chromium, cobalt-chromium alloys, etc. and various flexible materials such as ethylene-tetrafluoroethylene copolymer (ETFE), polyurethane, polyether-nylon resins, etc.

In addition, the outer surface of the puncture needle or the puncture body is preferably formed with grooves so that echoes are reflected thereon. This ensures that puncture can be made while confirming the position of the puncture needle under echographic observation.

The shape of the puncture needle may be any of a rectilinear shape, a crescent shape, a fishhook-like shape, and a U-shape.

Further, the intermediate connection section is preferably in the shape of a linear body having a Luer lock-shaped joint which can be attached and detached, or in the shape of a linear body having a male-female screws (a pair of screws) which can be attached and detached by a torsion of pressure feed tube body itself, or in the shape of a wire which can be cut by a shears device or the like.

The properties such as the size, length, material and the like of the expansion body and the filling material used to the puncture-type expansion device of the present invention are the same as those in the expansion body of the expansion device assembly between spinous processes, and, therefore, descriptions of these are omitted here.

Another embodiment of the puncture-type expansion body device pertaining to the present invention preferably includes; a hollow puncture needle provided with a solid puncture body at the distal end thereof; a balloon-formed expansion body which is expanded by injecting a filling material into the inside thereof; an intermediate connection section which is provided between the puncture needle and the expansion body, and by which the expansion body is guided to a part punctured by the puncture needle; wherein, the puncture needle has a hollow containment portion at the proximal end thereof, and the intermediate connection section and the expansion body are located with being housed in the containment portion. In addition, the intermediate connection section is preferably a linear body which is attached to the puncture needle at one end thereof and to the expansion body at the other end thereof.

The puncture-type expansion body device is preferably a puncture-type expansion body device including: a hollow puncture needle 3 provided with a solid puncture body 2 at the distal end thereof; a balloon-formed expansion body 24 which is expanded by injecting a filling material into the inside thereof; an intermediate connection section which is provided between the puncture needle and the expansion body, is attached to the puncture needle at one end thereof and to the expansion body at the other end thereof, and has a linear body contained in the puncture needle and by which the expansion body is guided to a part punctured by the puncture needle; and a pressure feed tube body for injecting a filling material into the expansion body in the condition where the linear body and the expansion body are pulled out. Further, the above-mentioned pressure feeder or the like may be connected to the pressure feed tube body.

As a preferred embodiment of the puncture-type expansion device of the present invention, Fig. 4B is a pattern diagram of a preferred embodiment of the puncture-type expansion device. In Fig. 4B, the device has an expansion body 24 formed by a plurality of bloated parts interconnected via constricted parts, pressure feed tube body 26 for injecting a filling material, a linear intermediate connection section 4 for guiding the expanding body. An inlet (not shown) is formed on the expansion body 24 and the pressure feed tube body 26 is connected to the inlet. Further, the pressure feed tube body 26 is connected with the pressure feeder such as a syringe, a pump and so on, and the other edge of the inlet is connected with the puncture body 3 via the intermediate connection section 4 (see Fig. 4). In addition, an internal space with opened system is formed at the proximal end of the puncture needle, and the internal space is capable of housing the expansion body 24 as a containment portion. The expansion body 24 is folded and housed in the containment portion which is located inside of the puncture body 3 at regular state and, since the pressure feed tube body 26 for injecting the filling material and the expansion body 24 are interconnected, the expansion body 24 can be extracted outside within the range of the length of the intermediate connection section 4 by pulling the pressure feed tube body 26 toward the distal side. Moreover, the expansion body 24 is a hollow balloon with an internal space and there is a closed system except for the inlet, thereby the lumen inside the pressure feed tube body 26 and the inside of the expansion body are interconnected.

The expansion body 24 is preferably expanded to the predetermined size in the condition where the expansion body is extracted together with the linear body from the containment portion.

The expansion body has a structure having a plurality of bulging parts interconnected through a constricted part when expanded and the puncture needle is preferably fishhook-shaped, suture needle-shaped, crescent-shaped or U-shaped. Further, the intermediate connection section 4 is preferably in the shape of a linear body having a Luer lock-shaped joint which can be attached and detached, or in the shape of a linear body having a male-female screws (a pair of screws) which can be attached and detached by a torsion of pressure feed tube body itself, or in the shape of a wire which can be cut by a shears device or the like. Or, the expansion body may be contained inside the puncture needle. In this case, the puncture needle and the expansion body are preferably interconnected through a wire or the like which can be cut by a shears device or the like. Further, in the case where the expansion body is contained inside the puncture needle, the inside diameter of the puncture needle at the proximal end of the puncture-type expansion device is required about 0.5 to 9 mm.

A method for dilating between spinous processes used the expansion device assembly between spinous processes and the puncture-type expansion body device pertaining to the present invention will be described below.

Both of the expansion device assembly between spinous processes and the puncture-type expansion body device according to the present invention include a filamentous body provided with an expansion body. For instance, in the case of the expansion device assembly between spinous processes, the filamentous body provided with the expansion body refers to a filamentous body provided with the expansion body which includes a plurality of bulging parts interconnected through a constricted part. In the case of the puncture-type expansion body device, the filamentous body provided with the expansion body refers to a puncture-type expansion body device itself. A method for dilating between spinous processes used the expansion device assembly between spinous processes and the puncture-type expansion body device comprises, preferably, percutaneously inserting a filamentous body provided with an expansion body to locate the expansion body between the spinous processes (Step I), injecting a filling material into the expansion body to dilate between the spinous processes (Step II), and cutting off the expansion body from the filamentous body to locate the expansion body between the spinous processes (Step III).

Now, preferred embodiments of the present invention will be described below, divisionally referring to Steps I to III.

### (Step I)

First, in Step I, the filamentous body provided with the expansion body is percutaneously inserted to locate the expansion body between spinous processes. Specifically, it is preferable that the filamentous body provided with the expansion body is percutaneously inserted so as to penetrate the interspinal ligament between the adjacent spinous processes in a direction crossing the axial direction of the spinal column, and that the expansion body is located in the interspinal ligament between the spinous processes. More preferably, an axial-directionally central portion of the expansion body is located in the interspinal ligament between the spinous processes.

The method of locating the filamentous body provided with the expansion body between the spinous processes is not specifically restricted. Specific examples of the locating method include the following three methods: (1) a method in which a puncture needle having at least one lumen is percutaneously introduced into the vicinity of an interspinal ligament, the interspinal ligament is incised by a shears device inserted and passed through the lumen of the puncture needle, and thereafter the filamentous body provided with the expansion body at the distal end thereof is percutaneously inserted through the lumen to locate the expansion body between the spinous processes; (2) a method in which a puncture needle having at least one lumen is percutaneously introduced into the vicinity of an interspinal ligament, the interspinal ligament is incised by the puncture needle, and thereafter the filamentous body provided with the expansion body at the distal end thereof is inserted into and located in an introduction site in the interspinal ligament; and (3) a method in which a filamentous body provided with a puncture-type expansion body including a puncture needle at the distal end thereof and being connected to an expansion body through an intermediate connection section, or having an expansion body contained in the puncture needle, with the each expansion body being in connection with a pressure feed tube body, is used, and the filamentous body is made to directly penetrate the skin and the interspinal ligament between the spinous processes in a direction crossing the axial direction of the spinal column to locate the expansion body between the spinous processes. The three methods will be described in detail below.

(1) Step I of locating the filamentous body provided with the expansion body between spinous processes according to the present invention is preferably carried out in such a manner that the puncture needle having at least one lumen is percutaneously introduced into the vicinity of the interspinal ligament or into the interspinal ligament, the interspinal ligament is incised by a shears device inserted and passed through the lumen of the puncture needle, and thereafter the filamentous body provided with the expansion body at the distal end thereof is percutaneously inserted through the lumen to locate the expansion body between the spinous processes.

Specifically, in the method of (1) above, which is used the expansion device assembly between spinous processes, the puncture needle having at least one lumen is made to percutaneously puncture, for example, a lumbar part to be treated so as to bring the distal end of the puncture needle into the vicinity of an interspinal ligament or into the interspinal ligament, then a shears device is inserted and passed through the lumen of the puncture needle, and, further, the interspinal ligament between the adjacent spinous processes is incised by the shears device so as to penetrate the interspinal ligament in a direction crossing the axial direction of the spinal column. Next, the filamentous body provided with the expansion body at the distal end thereof is inserted into the lumen or another lumen. In this case, if the filamentous body and the shears device are inserted in the same lumen, the shears device is pulled out of the lumen, as required. Further, the expansion body is inserted and passed through the penetratingly incised part, and the expansion body is interposed within the interspinal ligament, whereby the expansion body is located between the spinous processes.

Besides, a plurality of puncture needles each having at least one lumen may be used. In this case, a process may be adopted in which at least two puncture needles each having at least one lumen are percutaneously introduced into the vicinity of the interspinal ligament in the same manner as above-mentioned, then a shears device is inserted into a lumen of one of the puncture needles, the interspinal ligament is incised by the shears device, and thereafter the filamentous body provided with the expansion body at the distal end thereof is passed through a lumen of other puncture needle to be inserted into and left in the incised part in the interspinal ligament.

Another process may be adopted in which the puncture needle having at least one lumen is made to puncture a body surface from a direction inclined against the body surface, the distal end of the puncture needle is brought into the vicinity of the interspinal ligament, an inner needle is evulsed, then the shears device is inserted and passed through the lumen of the puncture needle, the interspinal ligament between the adjacent spinous processes is incised by the shears device so as to penetrate the interspinal ligament in a direction crossing the axial direction of the spinal column to provide a through-hole, and thereafter the expansion body is inserted and passed through the through-hole by way of the lumen. Furthermore, a further process may be adopted in which the puncture needle having at least one lumen is made to puncture a body surface in such a manner as to be orthogonal to the axial direction of the spinal column (or directly above a predetermined interspinal ligament), the inner needle is evulsed, the shears device is inserted and passed through the lumen of the puncture needle, then the interspinal ligament is incised by the shears device in a left-right direction to provide the interspinal ligament with a T-shaped through-hole, and thereafter the expansion body is inserted and passed through the through-hole by way of the lumen.

(2) Step I of disposing the filamentous body provided with the expansion body between spinous processes according to the present invention is preferably carried out in such a manner that the puncture needle having at least one lumen is percutaneously introduced into the vicinity of an interspinal ligament, the interspinal ligament is penetrated by the puncture needle in a direction crossing the axial direction of the spinal column, and thereafter the filamentous body provided with the expansion body at the distal end thereof is inserted and passed through the penetrated part in the interspinal ligament by way of the lumen.

Specifically, the method of the above paragraph (2), which is used the expansion device assembly between spinous processes, differs from the method of the above paragraph (1) in that the interspinal ligament between spinous processes is penetrated by the puncture needle itself in a direction crossing the axial direction of the spinal column, without use of the shears device. Specifically, the puncture needle having at least one lumen is made to percutaneously puncture, for example, a lumbar part to be treated, and the interspinal ligament between the predetermined adjacent spinous processes is penetrated by the puncture needle in a direction crossing the axial direction of the spinal column. Then, the filamentous body provided with the expansion body at the distal end thereof is inserted into the lumen of the puncture needle, the expansion body is inserted and passed through the penetrated part, and the expansion body is interposed in the interspinal ligament to be located between the spinous processes.

After the puncture needle is percutaneously introduced into the vicinity of the interspinal ligament and the interspinal ligament is incised with the shears device or the puncture needle and before the filamentous body provided with the expansion body is percutaneously inserted through the lumen of the puncture needle, a filamentous body provided with a second expansion body different from the above-mentioned expansion body may be percutaneously inserted and expanded in a provisional manner. Preferably, the second expansion body can be expanded mainly by use of a liquid at a high pressure. As the liquid for expansion, for example, physiological saline or a radiopaque contrast agent or the like can be used. With the second expansion body thus preliminarily expanded at a high pressure, a space for expansion of the above-mentioned expansion body upon injection of the filling material into the inside of the above-mentioned expansion body in Step II described later can be sufficiently formed in a provisional manner. The second expansion body may be a balloon-formed one similar to the above-mentioned expansion body, or may be one that is expanded by a mechanical mechanism. The mechanical mechanism here may, for example, be one that uses an expansively deformable structure such as a freely deformable cage woven knitted a shape memory alloy in a linear form.

(3) Step I of locating the filamentous body provided with the expansion body between spinous processes according to the present invention is preferably a technique carried out by use of the puncture-type expansion body device pertaining to the present invention. Specifically, Step I is preferably a method in which the puncture-type expansion body device is made to directly penetrate a skin and an interspinal ligament between spinous processes in a direction crossing the axial direction of the spinal column, and, after pulling the puncture needle out of the living body, the puncture needle or the intermediate connection section and the pressure feed tube body are gripped and slid in the axial direction to locate the expansion body between the spinal processes.

Thus, the method of the above paragraph (3) is a technique carried out by use of the puncture-type expansion body device in which the filamentous body provided integrally with the expansion body, the shears device and the puncture needle in the method of the above paragraph (1). Specifically, the method of is a method in which the puncture-type expansion body device is made to perform percutaneous puncture, an interspinal ligament is penetrated by the puncture needle in a direction crossing the axial direction of the spinal column, and the puncture needle is pulled out of the living body, whereby the expansion body is located between spinous processes and in the interspinal ligament. More specifically, after the puncture-type expansion body device is made to puncture, for example, a lumbar part to be treated, the puncture needle is made to penetrate the interspinal ligament between the predetermined adjacent spinous processes in a direction crossing the axial direction of the spinal column, the puncture needle is pulled out from the predetermined position to the outside of the living body, and, further, the puncture needle or the intermediate connection section and the pressure feed tube body are gripped and slid in the axial direction of the puncture needle, whereby the expansion body is located between the spinous processes and in the interspinal ligament.

### (Step II)

Next, in Step II, the filling material is injected into the expansion body to dilate between the spinous processes. Specifically, when the filling material is injected into the inside of the expansion body located between the spinous processes, the expansion body is expanded to thereby dilate between the spinous processes.

More specifically, the expansion body of the expansion device assembly between spinous processes and the puncture-type expansion body device in the present invention is provided with an injection port, and the pressure feed tube body communicates with the injection port. Further, a pressure feeder such as a syringe and a pump is attached to the pressure feed tube body. When the filling material is added to the pressure feeder, such as a syringe or a pump, and the pressure feed tube body and the pressure feeder is operated, the filling material is injected through the pressure feed tube body into the expansion body, whereby the expansion body can be expanded.

In addition, after Step I and before the injection of the filling material into the expansion body, it is preferable to inject a (radiopaque) contrast agent into the expansion body inserted into and located in a incised part in an interspinal ligament, to confirm the position of the expansion body under radioscopy and then to discharge the contrast agent.

This makes it possible to confirm that the expansion body is located at a desired position.

The contrast agent is not specifically restricted, insofar as it is opaque to radioactive rays; thus, known radiopaque materials can be used. Specific examples of the radiopaque material include: iodine, barium, bismuth, boron, bromine, calcium, gold, platinum, silver, iron, manganese, nickel, gadolinium, dysprosium, tungsten, tantalum, stainless steel, Nitinol, their compounds such as barium sulfate, etc. and their solutions and dispersions (e.g., physiological saline); amidotrizoic acid (3,5-diacetamino-2,4,6-triiodobenzoic acid), sodium meglumine amidotrizoate, meglumine amidotrizoate, sodium iotalamate, meglumine iotalamate, meglumine iotroxate, iotrolan, ioxaglic acid, ioxilan, iopamidol, iopromide, iohexol, ioversal, iomeprol; ethyl ester of poppy oil fatty acid iodide (e.g., Lipiodol™, which is poppyseed oil in which a carbon atom is iodized). These radiopaque materials may be used either singly or in the form of a mixture of two or more of them. Or, alternatively, a contrast agent layer containing the above-mentioned contrast agent as a base material may be provided on the expansion body.

It suffices that the contrast agent is injected in such an amount as to make it possible to confirm that the expansion body is located at a desired position in an interspinal ligament. Normally, the injection amount of the contrast agent may be about 1 to 180 mL.

### (Step III)

In Step III, the expansion body is cut off from the filamentous body to be located between the spinous processes. Specifically, after the expansion body is located in the interspinal ligament between the spinous processes and then the filling material is injected into the expansion body to expand the expansion body in Step I and Step II, Step III is carried out in which the expansion body expanded by injection of the filling material thereinto is cut off from the filamentous body to be located between the spinous processes.

The state at which the expanded expansion body is cut off from the filamentous body is appropriately selected according to such factors as the filling material used and the method adopted for Step I. In relation to this technique, examples of the situation include the following four cases: (4) the case of using the hardening material being fluid at the time of injection and hardening after the injection; (5) the case of using the material being fluid at the time of injection and non-hardening after the injection; (6) the case of using the filamentous body having the puncture-type expansion body in Step I; and (7) the case of using non-fluid material (including cases of co-use with a fluid material material) as the filling material. Now, the technique in these three cases will be described below.

### (4) The case of using the hardening material being fluid at the time of injection and hardening after the injection

It is preferable that in Step III, before the expansion body is cut off from the filamentous body to be located between the spinous processes, the expansion body is expanded by injection of the filling material thereinto, then hardening of the hardening material to be solid or gel is waited for, after which the expansion body portion near the injection port of the expansion body is resected by the shears device to cut the expansion body off the filamentous body, and the expanded expansion body is thereby located between the spinous processes. Besides, this procedure is preferably used in the case where a plurality of (for example, two) puncture needles each having at least one lumen are made to puncture a living body.

In the case where a single puncture needle is made to puncture a living body, another procedure that the expansion body cut off from the filamentous body to locate the expansion body between the spinous processes are used. Examples of the another procedure include: (A) a procedure in which the shears device is inserted and passed through another lumen of the puncture needle and an expansion body portion near the injection port is resected; (B) a procedure in which for resection of an expansion body portion near the injection port of the expansion body, a puncture needle having a lumen is newly made to percutaneously puncture, the shears device is inserted and passed through this lumen, the expansion body is cut off inside the living body, and the expanded expansion body is thereby located; (C) a procedure in which for inustion of an expansion body portion near the injection port of the expansion body, a puncture needle having a lumen is newly made to percutaneously puncture, an inustion wire is inserted and passed through this lumen, the expansion body is cut off inside the living body by inustion, and the expanded expansion body is thereby located; (D) a procedure in which after the hardening material is injected into the expansion body to expand the expansion body, the injection port of the expansion body is pulled out of the living body, the injection port of the expansion body thus pulled out is cut off, and the injection port is embedded subcutaneously; (E) a procedure in which for resection of an expansion body portion near the injection port of the expansion body, the shears device is inserted and passed through the lumen of the already inserted puncture needle so as to locate the pressure feed tube body of the expansion body along it, the expansion body is cut off inside the living body, and the expanded expansion body is thereby located; (F) a procedure in which a shears mechanism is already assembled in the inside of the puncture needle near its distal end, and, for resection of an expansion body portion near the injection port of the expansion body in a remote manner, the shears mechanism is operated from a puncture needle proximal portion outside the living body to cut off the expansion body inside the living body, and the expanded expansion body is thereby located; and (G) a procedure in which joint parts (for example, male-female screws, Luer lock, etc.) are already installed at the expansion body and the pressure feed tube body, and, for resection of an expansion body portion near the injection port of the expansion body, a pressure feed tube body portion located outside the living body is twisted to cut off the joint parts of the expansion body and the pressure feed tube body which are located inside the living body, and the expanded expansion body is thereby located.

Yet another method is (H) a procedure in which as the filamentous body provided with an expansion body at the distal end thereof in the present invention, the one-way valve described in PCT Patent Publication WO2008/11107 is attached to the expansion body, whereby the expansion body can be easily separated from the filamentous body.

The shears mechanism includes a mechanism which mainly generate shearing force, such as scissors, cutter and so on.

### (5) The case of using the material being fluid at the time of injection and non-hardening after the injection

In the method in which the expansion body is cut off from the filamentous body to locate the expansion body between the spinous processes, examples of the method include: (I) a method in which after the non-hardening material is injected into the expansion body to expand the expansion body, the injection port of the expansion body is sealed off, and the expansion body is cut off to be located; (J) a method in which after the non-hardening material injected into the expansion body to expand the expansion body, the injection port of the expansion body is pulled out of the living body, and the injection port is bundled by a fastening member, to cut off the expansion body in the outside of the living body, and the injection port is embedded subcutaneously; and (H) the method in which as the filamentous body provided with an expansion body at the distal end thereof in the present invention, the one-way valve described in PCT Patent Publication WO2008/11107 is attached to the expansion body.

### (6) The case of using the filamentous body provided with the puncture-type expansion body of Step I

A method in which is cutting off the expansion body from the filamentous body to leave the expansion body between the spinous processes includes the following procedure (K) and (L). Examples of the method applicable in this case include a method in which the method of (1) above is adopted in carrying out Step I, and, after Step II is conducted, in the case where the intermediate connection section is a linear body having a Luer lock-shaped joint, after the puncture-type expansion body device is made to directly penetrate the skin and the interspinal ligament between the spinous processes in a direction crossing the axial direction of spinal column and the puncture needle is pulled out of the living body, the intermediate connection section is separated from the expansion body by twisting, the pressure feed tube body is gripped and is slid in the axial direction to locate the expansion body between the spinous processes, the expansion body is expanded by injection of the filling material, and thereafter the expansion body and the pressure feed tube body are separated by any of the above-mentioned procedures (A) to (J)(procedure (K)).

In addition, examples of the method applicable in this case include a methods in which the method of (1) above is adopted in carrying out Step I, and, after Step II is conducted, in the case where the intermediate connection section is wire-like in shape, after the puncture-type expansion body device is made to directly penetrate the skin and the interspinal ligament between the spinous processes in a direction crossing the axial direction of the spinal column and the puncture needle is pulled out of the living body, the intermediate connection section and the pressure feed tube body are gripped and slid in the axial direction of the puncture needle to locate the expansion body between the spinous processes, the expansion body is expanded by injection of the filling material, thereafter the intermediate connection section is cut to separate the puncture needle, and the expansion body and the pressure feed tube body are separated by any of the above-mentioned procedures (A) to (J) (procedure (L)).

### (7) The case of using a non-fluid material as the filling material (including cases of co-use with a fluid material)

A method for separating the expansion body from the filament body and making it locating between the spinous processes includes a method of inflating the expansion body by the injection of a filling material including a non-fluid material before separating the expansion body from the filament body to be located between the spinous processes in Step III and then, when a hardening material is co-used, curing the hardening material and waiting until the material becomes a solid or a gel state, and separating the expansion body and the pressure feed tube body by the method (A) to (H), and (K) and (L) mentioned above. In addition, when a material which cannot be hardened is co-used, a method of separating the expansion body and the pressure feed tube body by the method (I) to (L) mentioned above is included. Incidentally, after Step III, the puncture needle and the shears device and the like are evulsed, and stanching of the punctured part is conducted using a hemostatic means such as an adhesive bandage, a stapler, etc.

### EXAMPLES

Now, the method for dilating between spinous processes according to the present invention will be described in detail below, based on particularly preferred embodiments. It should be noted, however, that the technical scope of the present invention is not to be limited only to the following examples.

### Example 1

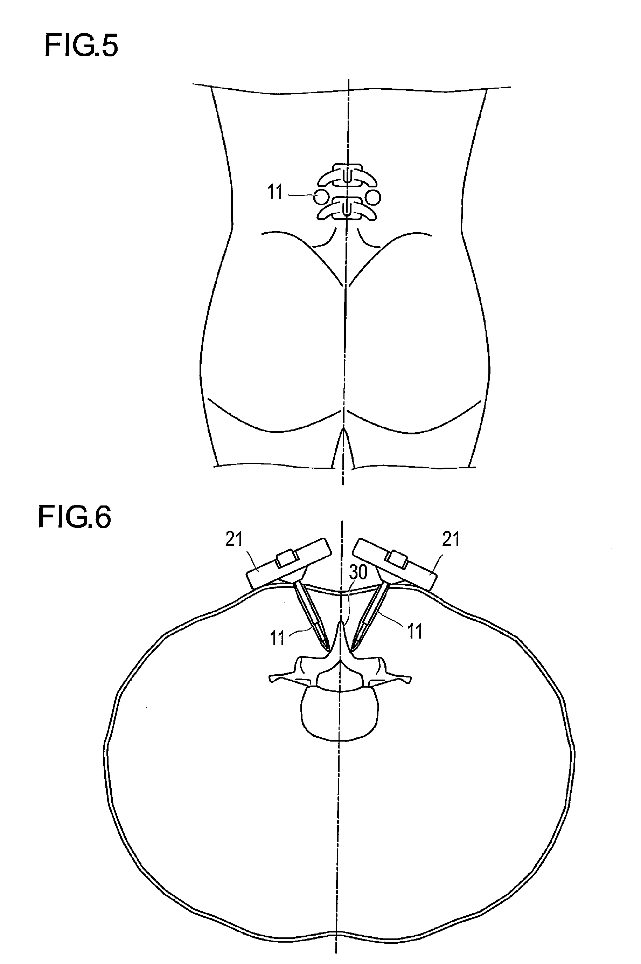

First, after the lesion is determined by roentgen or MRI technique, the part to be treated is anesthetized by local anesthesia or lumbar anesthesia. Then, the patient is put into prone position or lateral position, and the body is bent as much as possible, whereby the area between spinous processes 30 on the rear side of the vertebral body 33 and the intervertebral disk 32 is widened. Next, an approach site 11 for locating the expansion body is marked on the upper skin corresponding to the part between the spinous processes 30 which is the part to be treated (FIG. 5). Two puncture needles 21 having an inside diameter of 1 to 5 mm are made to penetrate the marked part by about 1 to 2 cm from the skin until they reach the paravertebral muscle. Then, the inner needles of the puncture needles 21 are pulled out, and the outer tubes of the puncture needles 21 are put locateing (FIG. 6).

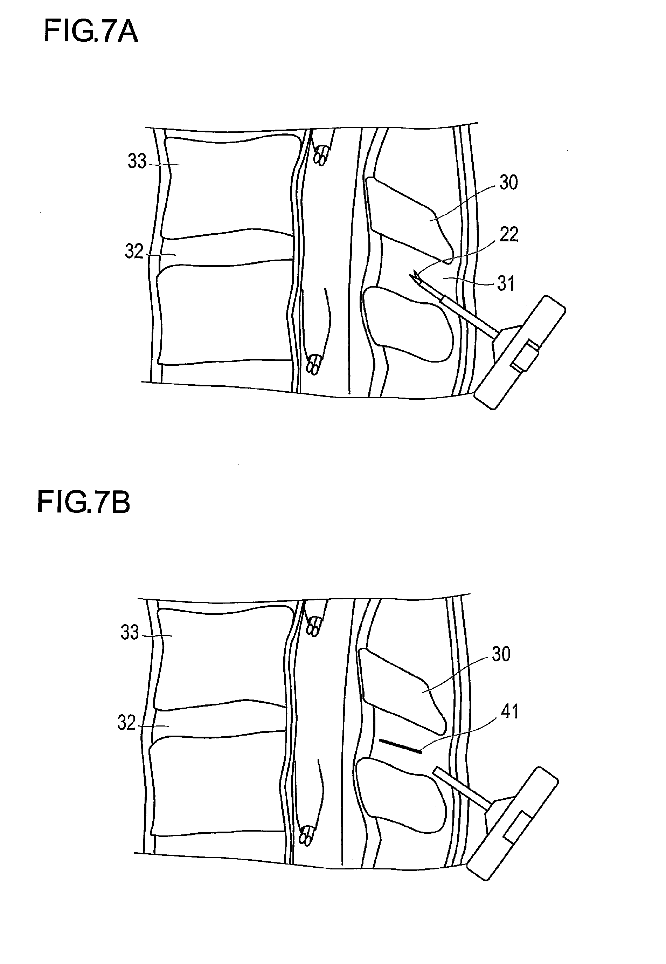

Rongeurs 22 as shears devices are inserted respectively into the lumens of the two puncture needles 21 (FIG. 7A). While holding down the puncture needles 21 by use of the rongeurs 22, cutting 41 of the interspinal ligament 31 to a depth of about 1 to 3 cm is conducted by use of the two rongeurs 22 (FIG. 7B). The cutting is conducted under radioscopic or echographic observation, with care not to damage the nerves by the rongeurs 22. In this instance, it is desirable to restrict the cut part 41 to within the interspinal ligament 31 so as not to damage the paravertebral muscles in the surroundings.

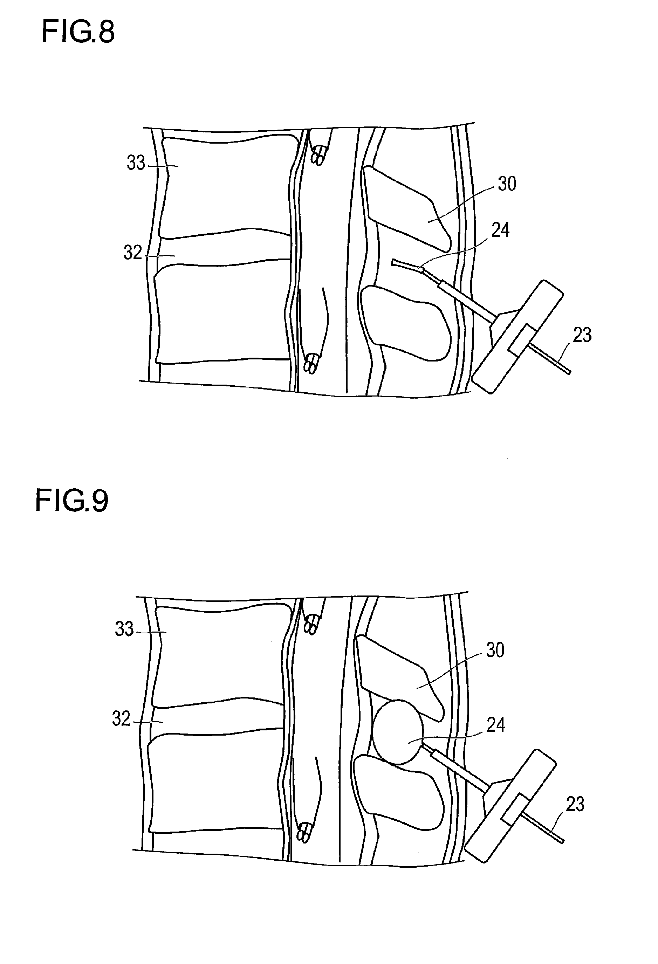

The rongeur 22 is then pulled out of the lumen of the puncture needle 21, and the filamentous body device 23 provided with the expansion body is inserted (FIG. 8). The puncture needle 21 on one side in which the rongeur 22 is not inserted is evulsed. The part where the puncture needle 21 has been evulsed is stanched.

A contrast agent is injected into the filamentous body 23 provided with the expansion body. The locateing site for the expansion body 24 is determined under radioscopic observation.

The expansion body 24 is preferably a dumbbell-shaped body, having a diameter and a length in the range of 1 to 5 cm when expanded. A proper position for the expansion body 24 is so set that a central portion of the expansion body 24 is located in the middle between the spinous processes 30 and that the expansion body 24 would not damage nerves or the like. When it is confirmed that the expansion body 24 is located in the interspinal ligament 31 and between the spinous processes 30, the contrast agent and the like are discharged from the expansion body 24 as much as possible. A hardening material being fluid at the time of injection and hardening after the injection, such as bone cement or polymer, is injected as a filling material into the filamentous body 23 provided with the expansion body, to expand the expansion body (FIG. 9). The pressure of expansion by injection of the filling material is set in the range of 10 to 50 atm. Under radioscopic observation, the expansion body is expanded to dilate between the spinous processes 30 by 1 to 5 cm to such an extent that antecurvature is maintained.

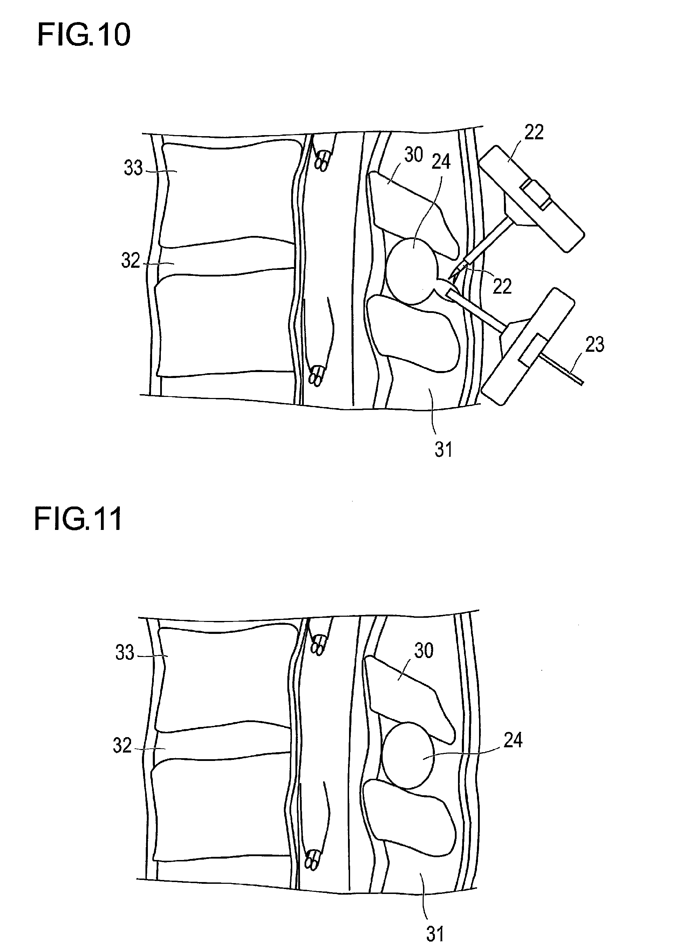

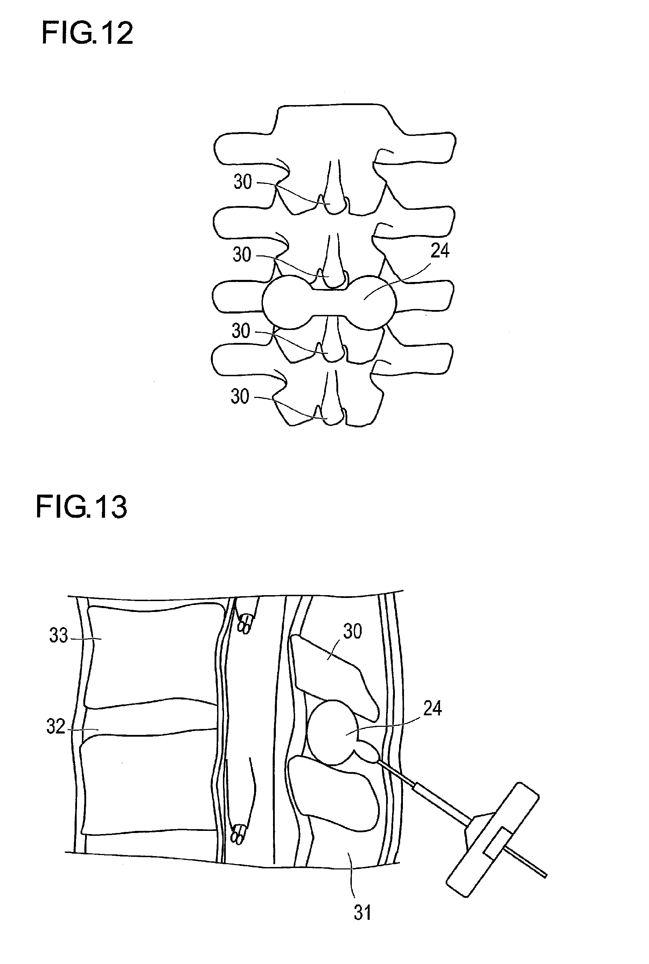

In the case where the filling material is a bone cement, the filling material injected is waited for to be hardened, and, after the bone cement is hardened, a puncture needle 21 is newly made to puncture at the side of the filamentous body 23 provided with the expansion body. Then, the inner needle of the puncture needle 21 is evulsed, and a rongeur 22 is inserted into the lumen of this puncture needle 21. From the filamentous body 23 provided with the expansion body, the expansion body 24 is cut off subcutaneously by use of the rongeur 22 (FIG. 10). Furthermore, the expansion body 24 is located in the interspinal ligament 31 and between the spinous processes 30 (FIGS. 11 and 12). The puncture needle 21, the rongeur 22 and a shaft 26 (or, the pressure feed tube body 26 and the pressure feeder 27) are evulsed, and the part where the puncture needle 21 has been evulsed is stanched.

### Example 2

After the same technique as in Example 1 is conducted and the contrast agent and the like are discharged from the expansion body 24 as much as possible, a hardening material being fluid at the time of injection and hardening after the injection is injected as a filling material into the filamentous body 23 provided with the expansion body, to expand the expansion body (FIG. 13).

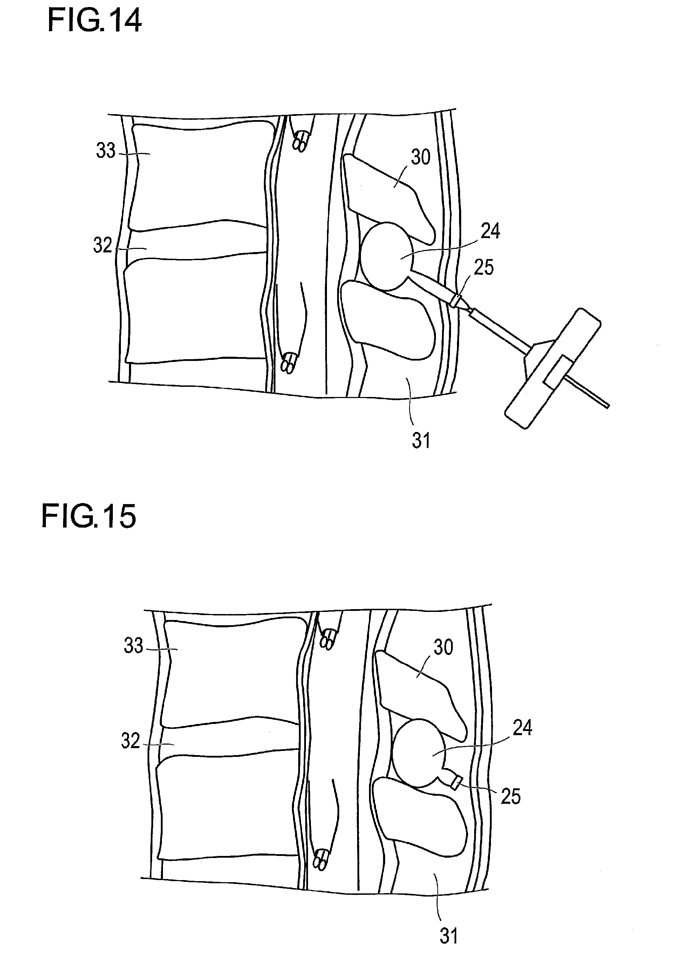

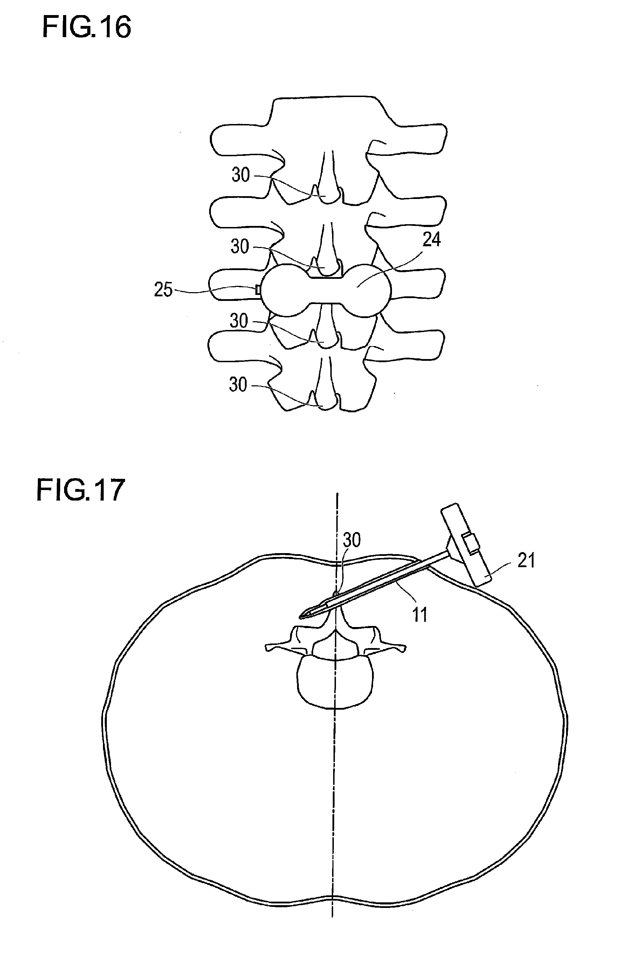

Next, the puncture needle 21 is evulsed from the skin, and the shaft 26 of the filamentous body 23 provided with the expansion body is exposed. The filamentous body 23 provided with the expansion body is pulled proximally to such an extent that the expansion body 24 is not pulled off, whereby the injection port 25 of the expansion body 24 is elongated by 0.2 to 1 cm. Then, the injection port 25 is tied with a twisted suture or a band or the like on the outside of the skin, to be sealed (FIG. 14). Further, the filamentous body portion on the proximal side of the seal is resected by use of scissors. The cut-off part is pushed to under the muscle layer by use of tweezers or a bar or the like, and the expansion body 24 is located between the spinous processes 30 (FIGS. 15 and 16). The part where the shaft 26 has been evulsed is stanched.

### Example 3

In the same manner as in Example 1, after the lesion is determined by roentgen or MRI technique, the part to be treated is anesthetized by local anesthesia or lumbar anesthesia, the patient is put into prone position or lateral position, and the body is bent as much as possible, to widen the area between the spinous processes 31. Next, marking of one approach site for placement of the expansion body is made on the upper skin corresponding to the part between the spinous processes 30 which is to be treated. A puncture needle 21 having an inside diameter of 1 to 5 mm is made to puncture the marked part to a depth of about 1 to 2 cm from the skin, until it reaches the paravertebral muscle 31. Then, the inner needle of the puncture needle 21 is pulled out, and the outer needle of the puncture needle 21 is located (FIG. 17).

A rongeur 22 is inserted into the lumen of the puncture needle 21. While holding down the puncture needle 21, cutting 41 of the interspinal ligament 31 to a depth of about 1 to 3 cm is conducted using the rongeur 22. The cutting is carried out under radioscopic or ecographic observation, with care not to damage nerves by the rongeur 22. In this instance, it is preferable to restrict the cut part 41 within the interspinal ligament 31, so as not to damage paravertebral muscles in the surroundings.

The rongeur 22 is evulsed from the lumen of the puncture needle 21, and the filamentous body 23 provided with the expansion body is inserted through the lumen.

A contrast agent is injected into the filamentous body 23 provided with the expansion body. The locating site for the expansion body 24 is confirmed under radioscopy.

The expansion body 24 is preferably dumbbell-shaped or H-shaped, with a diameter and a length in the range of 1 to 5 cm when expanded. A proper position for the expansion body 24 is so determined that a central portion of the expansion body 24 is located in the middle between the spinous processes 30 and that the expansion body 24 would not damage nerves or the like. When it is confirmed that the expansion body 24 is located in the interspinal ligament and between the spinous processes 30, the contrast agent and the like are discharged from the expansion body 24 as much as possible. A hardening material being fluid at the time of injection and hardening after the injection, such as bone cement or polymer, is injected as a filling material into the filamentous body 23 provided with the expansion body, to expand the expansion body. The pressure of expansion by injection of the filling material is set in the range of 10 to 50 atm. Under radioscopic observation, the expansion body is expanded to dilate between the spinous processes 30 by 1 to 5 cm to such an extent that antecurvature is maintained.

The bone cement as the filling material is waited for to be hardened, and, after the bone cement is hardened, a puncture needle 21 is newly made to puncture at the side of the filamentous body 23 provided with the expansion body. Then, the inner needle of the puncture needle 21 is evulsed, and a rongeur 22 is inserted into the lumen of this puncture needle 21. From the filamentous body 23 provided with the expansion body, the expansion body 24 is cut off subcutaneously by use of the rongeur 22. Further, the expansion body 24 is located between the spinous processes. The puncture needle 21, the rongeur 22 and a shaft (the pressure feed tube body and the pressure feeder) 26 are evulsed, and the part where the puncture needle 21 has been evulsed is stanched.

### Example 4

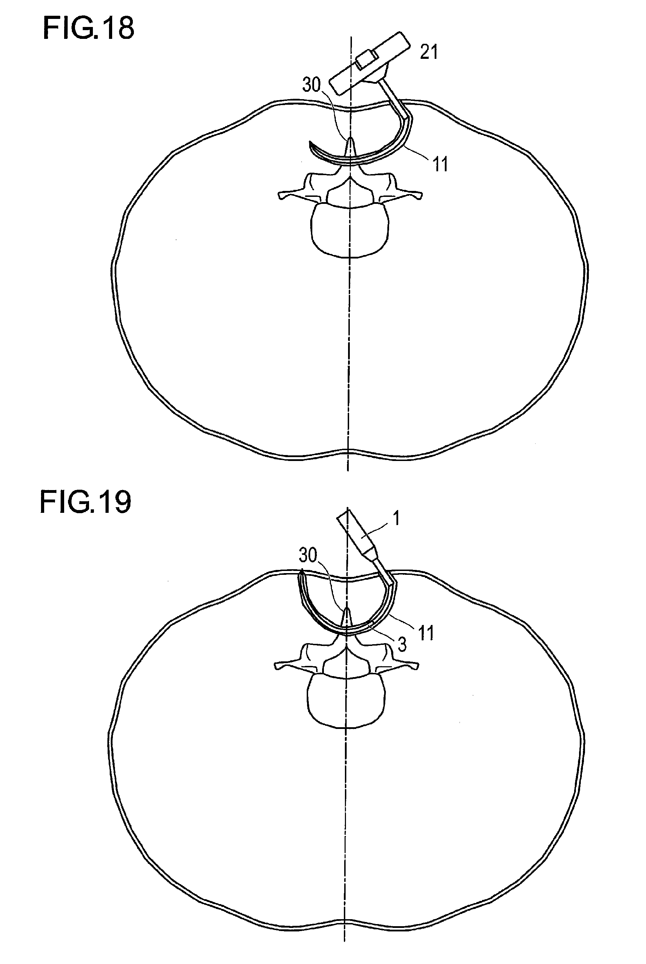

In the same manner as in Example 1, after the lesion is determined by roentgen or MRI technique, the part to be treated is anesthetized by local anesthesia or lumbar anesthesia, the patient is put into prone position or lateral position, and the body is bent as much as possible, to widen the area between the spinous processes 30. Next, marking of one approach site for placement of the expansion body is made on the upper skin corresponding to the part between the spinous processes 30 which is to be treated. Under radioscopy, a puncture needle 21 having an inside diameter of 1 to 5 mm and having a crescent-shaped sharp point part at the distal end is made to puncture the marked part to a depth of about 1 to 10 cm from the skin, so as to penetrate the interspinal ligament 31 between the spinous processes 30 in a direction crossing the axial direction of the spinal column (FIG. 18). In this instance, the part penetrated by the puncture needle is confirmed under radioscopy. Then, the filamentous body 23 provided with the expansion body is inserted through the lumen of the puncture needle 21 having the crescent-shaped sharp point part.

A contrast agent is injected into the filamentous body 23 provided with the expansion body. The locating site for the expansion body 24 is confirmed under radioscopy.

The dumbbell-shaped or H-shaped expansion body 24 has a diameter and a length in the range of 1 to 5 cm when expanded. The expansion body 24 is so disposed that a central portion thereof is disposed in the middle between the spinous processes 30, with care not to damage nerves or the like by the expansion body 24. When it is confirmed that the expansion body 24 is located in the interspinal ligament 31 and between the spinous processes 30, the contrast agent and the like are discharged from the expansion body 24 as much as possible. A hardening material being fluid at the time of injection and hardening after the injection, such as bone cement or polymer, is injected as a filling material into the filamentous body 23 provided with the expansion body, to expand the expansion body. The pressure of expansion by injection of the filling material is set in the range of 10 to 50 atm. Under radioscopy, the expansion body is expanded to dilate between the spinous processes 30 by 1 to 5 cm to such an extent that antecurvature is maintained.

The bone cement as the filling material is waited for to be hardened, and, after the bone cement is hardened, a shaft (the pressure feed tube body and the pressure feeder) 26 connected to the expansion body through a Luer lock is twisted, whereby the expansion body is cut off from the Luer lock part together with the hardened filling material, and is thereby located within the interspinal ligament 31 and between the spinous processes 30. Then, the puncture needle 21 and the shaft 26 are evulsed, and the part where the puncture needle 21 has been evulsed is stanched.

### Example 5

In the same manner as in Example 1, after the lesion is determined by roentgen or MRI technique, the part to be treated is anesthetized by local anesthesia or lumbar anesthesia, the patient is put into prone position or lateral position, and the body is bent as much as possible, to widen the area between the spinous processes 30. Next, marking of one approach site for placement of the expansion body is made on the upper skin corresponding to the part between the spinous processes 30 which is to be treated. Under radioscopy, a puncture needle 21 having an inside diameter of 1 to 5 mm and having a crescent-shaped sharp point part at the distal end is made to puncture the marked part to a depth of about 1 to 10 cm from the skin, so as to penetrate the interspinal ligament 31 between the spinous processes 30 in a direction crossing the axial direction of the spinal column (FIG. 18). In this instance, the part penetrated by the puncture needle is confirmed under radioscopy. Then, the filamentous body 23 provided with the expansion body is inserted through the lumen of the puncture needle 21 having the crescent-shaped sharp point part.

A (radiopaque) contrast agent is injected into the filamentous body 23 provided with the expansion body. The locating site for the expansion body 24 is confirmed under radioscopy.

The dumbbell-shaped or H-shaped expansion body 24 has a diameter and a length in the range of 1 to 5 cm when expanded. The expansion body 24 is so disposed that a central portion thereof is disposed in the middle between the spinous processes 30, with care not to damage nerves or the like by the expansion body 24. When it is confirmed that the expansion body 24 is located in the interspinal ligament 31 and between the spinous processes 30, the contrast agent and the like are discharged from the expansion body 24 as much as possible. A mixture of a two-part type crosslinking polymer such as a liquid silicone rubber and barium sulfate is injected as a filling material into the filamentous body 23 provided with the expansion body, to expand the expansion body. The pressure of expansion by injection of the filling material is set in the range of 10 to 50 atm. Under radioscopy, the expansion body is expanded to dilate between the spinous processes 30 by 1 to 5 cm to such an extent that antecurvature is maintained.

After the liquid silicone rubber as the filling material is hardened, the shaft (the pressure feed tube body and the pressure feeder) 26 connected to the expansion body through a one-way valve is cut off, and is thereby located in the interspinal ligament 31 and between the spinous processes 30. The puncture needle 21 and the shaft 26 are evulsed, and the part where the puncture needle 21 has been evulsed is stanched.

### Example 6

In the same manner as in Example 1, after the lesion is determined by roentgen or MRI technique, the part to be treated is anesthetized by local anesthesia or lumbar anesthesia, the patient is put into prone position or lateral position, and the body is bent as much as possible, to widen the area between the spinous processes 30. Next, marking of one approach site for placement of the expansion body is made on the upper skin corresponding to the part between the spinous processes 30 which is to be treated. Under radioscopy, a puncture needle 21 having an inside diameter of 1 to 5 mm and having a crescent-shaped sharp point part at the distal end is made to puncture the marked part to a depth of about 1 to 10 cm from the skin, so as to penetrate the interspinal ligament 31 between the spinous processes 30 in a direction crossing the axial direction of the spinal column (FIG. 18). In this instance, the part penetrated by the puncture needle is confirmed under radioscopy. Then, the filamentous body (not shown) provided with the expansion body is inserted through the lumen of the puncture needle 21 having the crescent-shaped sharp point part.

A contrast agent is injected into the filamentous body provided with the expansion body. The locating site for the expansion body is confirmed under radioscopy. The contrast agent and the like are discharged from the expansion body as much as possible.

Separately, a filamentous body 23 provided with a dumbbell-shaped or H-shaped expansion body 24 is inserted through the lumen of the puncture needle 21 having the crescent-shaped sharp point part. The expansion body 24 has a diameter and a length in the range of 1 to 5 cm when expanded. The expansion body 24 is so disposed that a central portion thereof is disposed in the middle between the spinous processes 30, with care not to damage nerves or the like by the expansion body 24. As the filling material, a spring coil made of stainless steel is inserted into the filamentous body 23 provided with the expansion body; thereafter, a hardening material being fluid at the time of injection and hardening after the injection, such as bone cement, is injected, to expand the expansion body. The pressure of expansion by injection of the filling material is set in the range of 10 to 50 atm. Under radioscopy, the expansion body is expanded to dilate between the spinous processes 30 by 1 to 5 cm to such an extent that antecurvature is maintained.

The bone cement as the filling material is waited for to be hardened, after which a shaft (the pressure feed tube body and the pressure feeder) 26 connected to the expansion body through a Luer lock is twisted, whereby the expansion body is cut off from the Luer lock part together with the hardened filling material, and is thereby located within the interspinal ligament 31 and between the spinous processes 30. Then, the puncture needle 21 and the shaft 26 are evulsed, and the part where the puncture needle 21 has been evulsed is stanched.

### Example 7

First, after the lesion is determined by roentgen or MRI technique, the part to be treated is anesthetized by local anesthesia or lumbar anesthesia. Then, the patient is put into prone position or lateral position, and the body is bent as much as possible, to widen the area between the spinous processes 30. Next, marking of one approach site for placement of the expansion body is made on the upper skin corresponding to the part between the spinous processes 30 which is to be treated. A puncture-type expansion body device provided with a puncture body with a fishhook-shaped distal end is made to puncture the marked part to a depth of about 1 to 20 cm from the skin, so as to penetrate the interspinal ligament 31 between the spinous processes 30 in a direction crossing the axial direction of the spinal column, and the puncture needle is pulled out of the living body. In this instance, the part penetrated by the puncture body is confirmed under radioscopy. Then, the puncture needle or the intermediate connection section for guiding the expansion body and a shaft portion of the pressure feed tube body on the proximal side are gripped and are slid forward or backward in the axial direction so that the expansion body is disposed in the interspinal ligament between the spinous processes (FIG. 19). Subsequently, after the locating site for the expansion body 24 is confirmed under radioscopy, a material being fluid at the time of injection and non-hardening after the injection is injected as a filling material by the pressure feeder, to expand the expansion body. Thereafter, in the condition where the injection port of the expansion body is pulled out of the living body, the injection port of the expansion body is fastened with a twisted suture on the outside of the living body, and separated off. Concurrently, the wire is cut to separate the puncture needle. Then, the fastened injection port is embedded subcutaneously, and the punctured part is stanched.

## Claims

1. A puncture-type expansion body device comprising:
a puncture needle provided with a solid puncture body at a distal end thereof;
a balloon-formed expansion body which is expanded by injecting a filling material into the inside thereof;
an intermediate connection section which is provided between the puncture needle and the expansion body and by which the expansion body is guided to a part punctured by the puncture needle; and
a pressure feed tube body for injecting the filling material into the expansion body.

2. A puncture-type expansion body device according to claim 1, wherein the puncture needle has a hollow containment portion at a proximal end thereof, and the intermediate connection section and the expansion body are located with being housed in the containment portion.

3. A puncture-type expansion body device according to claim 1 or 2, wherein the intermediate connection section is a linear body which is attached to the puncture needle at one end thereof and to the expansion body at the other end thereof.

4. A puncture-type expansion body device according to claim 3, wherein the expansion body is expanded to a predetermined size in the condition where the expansion body is extracted together with the linear body from the containment portion.
